# EUROPEAN PATENT APPLICATION

(11) **EP 1 462 527 A1**
(43) Date of publication of application: **29.09.2004**
(21) Application number: 03006943.9
(22) Date of filing: 26.03.2003
(51) Int. Cl.: C12Q 1/68

(54) **Novel markers for inflammatory bowel disease**

(71) Applicant: CONARIS research institute AG, 24118 Kiel (DE)
(72) Inventor: Costello, Christine, Dr. Universitätsklinik S-H, 24105 Kiel (DE); Ma, Nancy, Universitätsklinik Schleswig Holstein, 24105 Kiel (DE); Schreiber, Stefan, Dr, Universitätsklinik S-H, 24105 Kiel (DE); Seegert, Dirk, Dr. CONARIS research institute AG, 24118 Kiel (DE)
(74) Representative: Schüssler, Andrea, Dr.

(57) **Abstract**

Described is a diagnostic composition comprising nucleic acid molecules which are capable of specifically hybridizing to the mRNAs of genes showing abnormal gene expression associated with inflammatory bowel disease (e.g., Crohn's disease (CD) and ulcerative colitis (UC)). The use of said nucleic acid molecules for diagnosis of such diseases or a disposition to such diseases is also described.

SEQUENCE LISTING

<110>  Conaris Research Institute AG

<120>  Novel Markers for Inflammatory Bowel Disease

<130>  C 1023EP

<140>  EP 03006943.9
<141>  2003-03-26

<160>  49

<170>  PatentIn version 3.2

<210>  1
<211>  21
<212>  DNA
<213>  Artificial

<220>
<223>  Primer M13 forward

<400>  1
cgttgtaaaa cgacggccag t                                                21

<210>  2
<211>  21
<212>  DNA
<213>  Artificial

<220>
<223>  Primer M13 reverse

<400>  2
tttcacacag gaaacagcta t                                                21

<210>  3
<211>  23
<212>  DNA
<213>  Artificial

<220>
<223>  Primer

<400>  3
gacgttgtaa aacgacggcc agt                                              23

<210>  4
<211>  30
<212>  DNA
<213>  Artificial

<220>
<223>  Primer

<400>  4
ataacaattt cacacaggaa acagctatga                                       30

<210>  5
<211>  17
<212>  DNA
<213>  Artificial

<220>
<223>  Primer

<400>  5
agcctcgcct ttgccga                                                    17


<210>  6
<211>  15
<212>  DNA
<213>  Artificial

<220>
<223>  Primer

<400>  6
ctggtgcctg gggcg                                                      15


<210>  7
<211>  22
<212>  DNA
<213>  Artificial

<220>
<223>  ß-actin Probe

<400>  7
ccgccgcccg tccacacccg cc                                              22


<210>  8
<211>  19
<212>  DNA
<213>  Artificial

<220>
<223>  Primer

<400>  8
aggcggtgct tgttcctca                                                  19


<210>  9
<211>  24
<212>  DNA
<213>  Artificial

<220>
<223>  Primer

<400>  9
gttcgagaag atgatctgac tgcc                                            24


<210>  10
<211>  25
<212>  DNA
<213>  Artificial

<220>
<223>  TNF-a Probe

<400>  10
ccagagggaa gagttcccca gggac                                           25


<210>  11

<211>  23
<212>  DNA
<213>  Artificial

<220>
<223>  Primer

<400>  11
ggaattgaat gggtttgcta gaa                                              23


<210>  12
<211>  21
<212>  DNA
<213>  Artificial

<220>
<223>  Primer

<400>  12
tgtggatcct ggctagcaga c                                                21


<210>  13
<211>  36
<212>  DNA
<213>  Artificial

<220>
<223>  IL-8 Probe

<400>  13
tggaaatcct ggattttttt ctgttaaatc tggcaa                                36


<210>  14
<211>  19
<212>  DNA
<213>  Artificial

<220>
<223>  Primer

<400>  14
gtgcccccta gcagtaccg                                                   19


<210>  15
<211>  19
<212>  DNA
<213>  Artificial

<220>
<223>  Primer

<400>  15
gaagtggctg ccactgctg                                                   19


<210>  16
<211>  29
<212>  DNA
<213>  Artificial

<220>
<223>  Mucin Probe

<400>  16

tgagaaggtt tctgcaggta atggtggca 29

<210> 17
<211> 22
<212> DNA
<213> Artificial

<220>
<223> Primer

<400> 17
atgcgcacaa atcccttcta cc 22

<210> 18
<211> 24
<212> DNA
<213> Artificial

<220>
<223> Primer

<400> 18
tcctcagaaa gagcagcatc gata 24

<210> 19
<211> 30
<212> DNA
<213> Artificial

<220>
<223> MMP-1 Probe

<400> 19
acgaatttgc cgacagagat gaagtccggt 30

<210> 20
<211> 23
<212> DNA
<213> Artificial

<220>
<223> Primer

<400> 20
tgtagaaggc acaatatggg cac 23

<210> 21
<211> 23
<212> DNA
<213> Artificial

<220>
<223> Primer

<400> 21
agtcacttgt ctgttgcaca cga 23

<210> 22
<211> 29
<212> DNA
<213> Artificial

<220>
<223>  MMP-3 Probe

<400>  22
atgttcgttt tctcctgcct gtgctgtga                                          29


<210>  23
<211>  21
<212>  DNA
<213>  Artificial

<220>
<223>  Primer

<400>  23
ttctggcatc ctgttgttgc t                                                  21


<210>  24
<211>  19
<212>  DNA
<213>  Artificial

<220>
<223>  Primer

<400>  24
tgacgaggtc ggaattgca                                                     19


<210>  25
<211>  20
<212>  DNA
<213>  Artificial

<220>
<223>  TIMP-1

<400>  25
cccccagcag ggcctgcacc                                                    20


<210>  26
<211>  20
<212>  DNA
<213>  Artificial

<220>
<223>  Primer

<400>  26
cccggacatc ttggcaaagt                                                    20


<210>  27
<211>  23
<212>  DNA
<213>  Artificial

<220>
<223>  Primer

<400>  27
gacacacagg atgcacaggt cat                                                23


<210>  28

<211>  25
<212>  DNA
<213>  Artificial

<220>
<223>  ATP7B Probe

<400>  28
aatcaaccag aggcagtggc agccg                                          25


<210>  29
<211>  21
<212>  DNA
<213>  Artificial

<220>
<223>  Primer

<400>  29
gcagccggag aacaactaca a                                              21


<210>  30
<211>  19
<212>  DNA
<213>  Artificial

<220>
<223>  Primer

<400>  30
ctgctgccac ctgctcttg                                                 19


<210>  31
<211>  25
<212>  DNA
<213>  Artificial

<220>
<223>  IGHG3 Probe

<400>  31
tggactccga cggctccttc ttcct                                          25


<210>  32
<211>  21
<212>  DNA
<213>  Artificial

<220>
<223>  Primer

<400>  32
accaaggtgg aaatcaaacg a                                              21


<210>  33
<211>  21
<212>  DNA
<213>  Artificial

<220>
<223>  Primer

<400>  33

aggcacacaa cagaggcagt t                                                21

<210>  34
<211>  27
<212>  DNA
<213>  Artificial

<220>
<223>  IGKC Probe

<400>  34
accatctgtc ttcatcttcc cgccatc                                            27

<210>  35
<211>  21
<212>  DNA
<213>  Artificial

<220>
<223>  Primer

<400>  35
ctgtcccagc cttgacttcc t                                                  21

<210>  36
<211>  24
<212>  DNA
<213>  Artificial

<220>
<223>  Primer

<400>  36
aagcttttca gtgactctga tgga                                               24

<210>  37
<211>  24
<212>  DNA
<213>  Artificial

<220>
<223>  ARTEMIS/SCIDA Probe

<400>  37
ttctgttatg tgtgttgact ggga                                               24

<210>  38
<211>  22
<212>  DNA
<213>  Artificial

<220>
<223>  Primer

<400>  38
attcagctgc atcaggaatc ag                                                 22

<210>  39
<211>  20
<212>  DNA
<213>  Artificial

<220>
<223>  Primer

<400>  39
ggcacatggt acagcaccaa                                                    20


<210>  40
<211>  33
<212>  DNA
<213>  Artificial

<220>
<223>  EST (Hs. 364872) Probe

<400>  40
attagcagca ataacaaaac aggaacagca gct                                     33


<210>  41
<211>  22
<212>  DNA
<213>  Artificial

<220>
<223>  Primer

<400>  41
gtgtaccaac tgcctggagg at                                                 22


<210>  42
<211>  20
<212>  DNA
<213>  Artificial

<220>
<223>  Primer

<400>  42
accccggcac tatcaccata                                                    20


<210>  43
<211>  23
<212>  DNA
<213>  Artificial

<220>
<223>  Claudin 4 Probe

<400>  43
ccaagaccat gatcgtggcg gtc                                                23


<210>  44
<211>  18
<212>  DNA
<213>  Artificial

<220>
<223>  Primer

<400>  44
ggccaagtgc cagtttgc                                                      18


<210>  45

<211>  21
<212>  DNA
<213>  Artificial

<220>
<223>  Primer

<400>  45
ggccctggag gtagaacttg t                                                21


<210>  46
<211>  25
<212>  DNA
<213>  Artificial

<220>
<223>  ZFP36 Probe

<400>  46
tcgccacccc aaatacaaga cggaa                                            25


<210>  47
<211>  20
<212>  DNA
<213>  Artificial

<220>
<223>  Primer

<400>  47
tttccccagc ctctcatcac                                                  20


<210>  48
<211>  21
<212>  DNA
<213>  Artificial

<220>
<223>  Primer

<400>  48
acagtcaggt cctggcggta t                                                21


<210>  49
<211>  33
<212>  DNA
<213>  Artificial

<220>
<223>  Fast Kinase Probe

<400>  49
tgcaagtaca gtcacaagga cataatagct gag                                   33

## Description

The present invention relates to a diagnostic composition comprising nucleic acid molecules which are capable of specifically hybridizing to the mRNAs of genes showing abnormal gene expression associated with inflammatory bowel disease (e.g., Crohn's disease (CD) and ulcerative colitis (UC)). The present invention also relates to the use of said nucleic acid molecules for diagnosis of such diseases or a disposition to such diseases.

Crohn's Disease (CD) and ulcerative colitis (UC) are two main forms of inflammatory bowel disease (IBD) and can be distinguished by both clinical and histopathological features. Complications in these chronic relapsing disorders include an increased risk of developing adenocarcinoma of the colon. IBD has a lifetime prevalence of up to 0.5% in western populations and the age of disease onset is typically in the second or third decade. Although extensively studied, most of the factors that cause IBD remain unknown and the current understanding of disease pathogenesis suggests a complex interplay of both environmental and genetic factors. Interestingly, inhibition of single factors in the pathophysiological cascade, e.g. TNF-α, with a monoclonal antibody leads to a marked improvement in a subgroup of patients with refractory Crohn's disease. Therefore, identification of key players in disease pathophysiology could be helpful for diagnosis, could identify important therapeutic targets and, furthermore, improve our understanding of aetiopathology. Unfortunately, so far suitable markers for diagnosis of IBD or for the determination of the risk to develop IBD are not available.

Thus, the technical problem underlying the present invention is to provide means (or markers) for diagnosis of inflammatory bowel diseases like Crohn's Disease (CD) or ulcerative colitis (UC) or diagnosis of a disposition to said diseases.

The solution to said technical problem is achieved by providing the embodiments characterized in the claims. Based on biomathematical analysis, gene expression profiles of CD/UC and control samples were analysed. Novel transcripts relevant to IBD could be identified and the possible role of known (and unknown) genes in disease pathophysiology was evaluated. High density cDNA arrays of 33,792 clones spotted as PCR products (obtained from a large whole genom chip; Human UniGene set RZPD 1) were used. Individual variability between normal controls was found to be high and is therefore an important factor to consider when designing microarray studies. After implementing stringent filtering criteria, 465 and 209 transcripts differentially regulated in CD and UC, respectively, were identified. These transcripts fell into such functional categories as immune responses (XBP1, IL1R1 & 2 and IGKC), pathogenesis (HLA-DQA1, HLA-DRB1), physiological processes (claudin 4), cell adhesion (cadherin 11), and oncogenesis (AGR2 & CDKN1A). A large proportion of the genes identified were unannotated. Prediction of the protein structure of a subsection of these unknown differentially regulated genes indicated that some could have specific functions in pathophysiology.

A large amount of true biological variation between samples from different individuals was detected. When array results from the normal patient samples (n=11) were compared, approximately 20% of colonic mucosal gene expression was found to be highly variable. This is not surprising considering genetic diversity (SNPs), and the fact that limited functional diversity (e.g. through food) cannot be avoided when working with probands and patients. Thus, studies in human populations have to take normal variability into account when defining abnormalities in disease. This determined the present approach in that cDNA from controls were not pooled, but rather a series of expression arrays representing the control population was generated.

An unequal number of over-expressed or repressed genes in the IBD subtypes was obsverved. In CD, approximately 80% of differentially expressed genes were found to be down-regulated, compared with 30% of genes in UC. The majority of differentially regulated transcripts were found to be unique to each IBD, indicating that these two diseases are indeed quite different in their expression profiles.

Genes differentially regulated only in CD relate to some known pathophysiological functional abnormalities in this disease. These included genes involved in leukotriene biosyntheses, such as arachidonate 5-lipoxygenase (ALOX5) and ALOX5-activating protein (ALOX5AP), which are necessary for leukotriene synthesis, and leukotriene B4 receptor BLT2, a recently described receptor of LTB4. The repression of these genes in CD possibly represents an attempt to dampen down the abnormal inflammatory response so characteristic of this disease. Several interesting genes involved in aberrant metabolism included occludin (OCLN), which is a major integral membrane component of mammalian tight junctions.

Genes uniquely regulated in UC included calpain 1 (CAPN1) which is a member of a family of calcium-dependent, cysteine proteases which can cleave an array of cellular proteins, including nuclear factor B (NF-B) (Wang and Yuen, Trends Pharmacol. Sci. 15 (1994), 412-419). Cuzzocrea et al. (Gut 48 (2001), 478-488) described the potent anti-inflammatory effects that calpain inhibitor 1 has in the reduction of colon injury and polymorphonuclear neutrophil infiltration in a rat model of experimental colitis. In the study leading to the present invention, the up-regulation of different collagens was noted, particularly in UC. It was also found that the expression of matrix metalloproteinase (MMPs), a group of peptidases involved in the degradation of the extracellular matrix, is up-regulated in IBD.

Only 93 transcripts were found to be common to both diseases and indeed, many are known to represent events that are common to inflammation in general. The largest fold difference observed between normal and IBD tissues was found in the expression of immunoglobulins. However, the up-regulation of these genes appeared to be more pronounced in UC, a fact that was confirmed by TaqMan analysis. This might reflect secondary events such as activated B cells as described in earlier immunopathophysiology studies. Interestingly, down-regulation of SCIDA/ARTEMIS was observed. The protein encoded by this gene is involved in V(D)J recombination, a mechanism that ensures the somatic diversification of immunoglobulin and antigen T-cell receptor-encoding genes (Li et al., J. Immunol. 168 (2002), 6323-6329), and therefore may prove highly relevant in IBD. Other genes that can be classified as involved in the immune response were up-regulation of HLA-DRB3, which plays a pivotal role in antigen recognition by binding extra cellular peptides, and X-box binding protein 1, which encodes a transcription factor that regulates MHC class II genes by binding to a promoter element referred to as an X-box. Up-regulation of zinc finger protein 36 (ZFP36), a gene which inhibits macrophage TNF-α production, was also shown. Although never associated with IBD, this gene has previously been shown to be up-regulated in human bronchial epithelial cells in response to TNF-α and IL1β. A finding that reflects the disrupted nature of the mucosa is down-regulation of ATPase, ATP7B. This protein is essential for normal distribution of copper in human cells and mutations lead to copper accumulation in a number of tissues and to a severe multisystem disorder, known as Wilson's disease (Nanji et al., Am. J. Hum. Genet. 60 (1997), 1423-1429). IBD is associated with increased intestinal permeability indicating disturbance of the mucosal barrier but it remains unclear whether this event occurs before intestinal inflammation or is a result of the inflammatory process. The finding that family members of CD patients have increased intestinal permeability, even though they themselves do not have the disease, has generated some interest in the possibility that genes involved in maintaining the intestinal barrier function may prove a major causative factor. Therefore, a very interesting finding was the up-regulation of members of the claudin family of proteins, a finding that was more pronounced in CD. Cadherin 11 (type 2) which encodes an integral membrane protein that mediates calcium-dependent cell-cell adhesion was also found to be up-regulated in both forms of IBD, and may reflect an effort to maintain the tight junctions between epithelial cells.

Approximately 45% and 30% of differentially regulated genes identified in CD and UC respectively are, as of yet, unannotated. Some transcripts analysed (e.g. Hs.102943, Hs.364872), did not have sufficient information to be assigned a putative function. The differential expression of genes whose products may be structural protein such as collagens (Hs.23921 & Hs.283848), and proteins involved in degradation (Hs.30532) and apoptosis (Hs.245326) may be induced by the inflammatory process itself and thus may not be connected to the primary cause of the disease. A relatively novel cytoskeletal protein found up-regulated in IBD was synaptopodin 2 (myopodin), which has been shown to directly bind actin in myoblasts (Weins et al., J. Cell. Biol. 155 (2001), 393-404). In contrast, a more detailed analysis of these unknown genes indicated that some may have the potential to cause pathology. Potential pathology causing transcripts examined in more detail include two transcripts (Hs.285519 & Hs.236463). In particular, the human homolog KIAA1238 (Hs 236463) of the ribosomal protein S6 modification enzyme may be associated with colon cancer. This appears to be the human homologue of the bacterial S6 modification enzyme RimK family. It has been shown that many human ribosomal protein transcripts, including S6 ribosomal protein, are over-expressed in colorectal tumours and polyps and that deletion of the 40S ribosomal S6 protein inhibits cell proliferation. Additionally, the dysfunctional involvement of lysophosphatidic acid acyltransferases (Hs.280858) in lipid synthesis may affect the development of cancer and inflammation-associated diseases. One unknown transcript (Hs.352537) mapped in close proximity to the 3' UTR of ADAM 12 (a disintegrin and metalloproteinase 12 (meltrin alpha)), which is activated by copper and mediates several important processes such as TNF-α release, fertilisation and myoblast fusion.

In summary, the main findings of the study leading to the present invention point to novel, important molecules in abnormal immune regulation and the highly disturbed cell biology of the intestine in IBD pathogenesis. A host of novel gene findings is reported that should be implicated in IBD pathophysiology and pathogenesis. The results from this study indicate that normal biological individual variability is an important factor to consider when designing microarray studies with patient samples. Results indicate that there are fundamental differences in the gene expression patterns in the IBD subtypes, CD and UC. Many of the expressed genes represent known genes never before implicated in IBD and many unknown ESTs. To conclude, the gene expression profiles of the present study provide a sensitive diagnostic resource and might enable to describe the pathways involved in the pathophysiology of diseases like CD or UD. The genes identified, therefore, represent new valuable candidates as diagnostic or therapeutic targets.

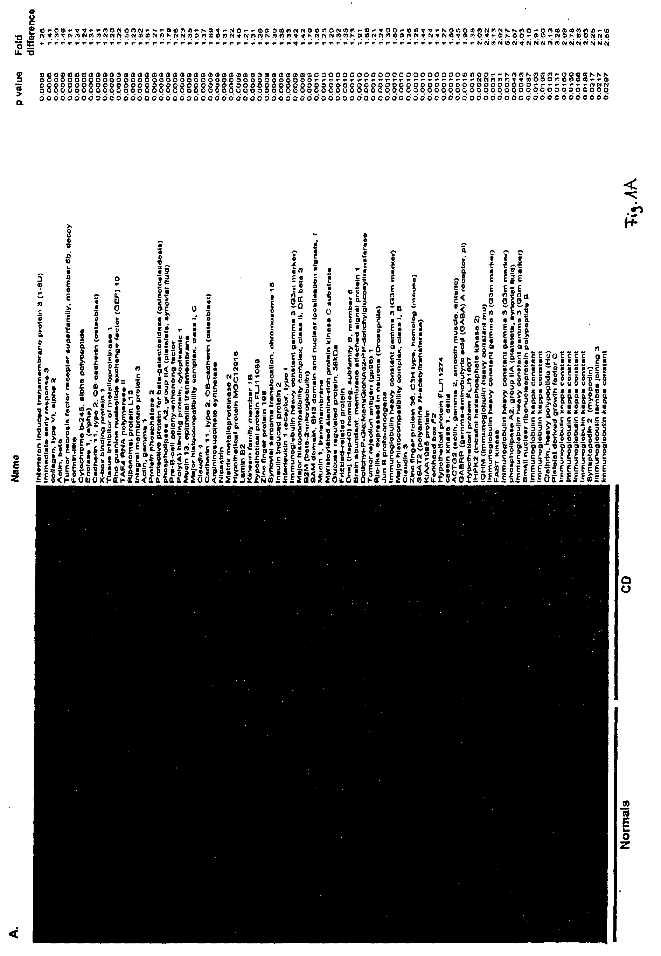

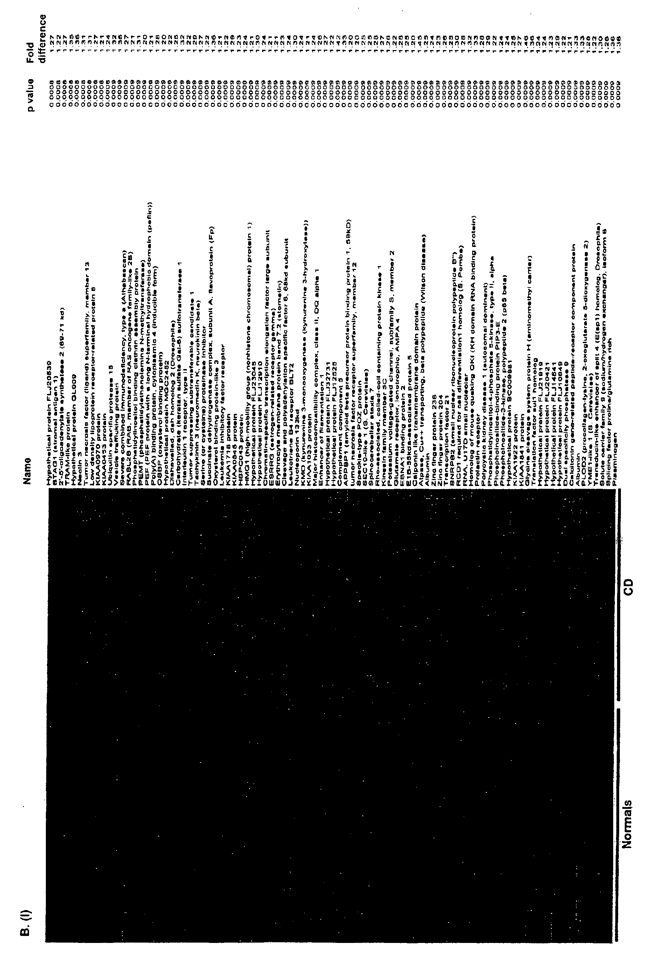

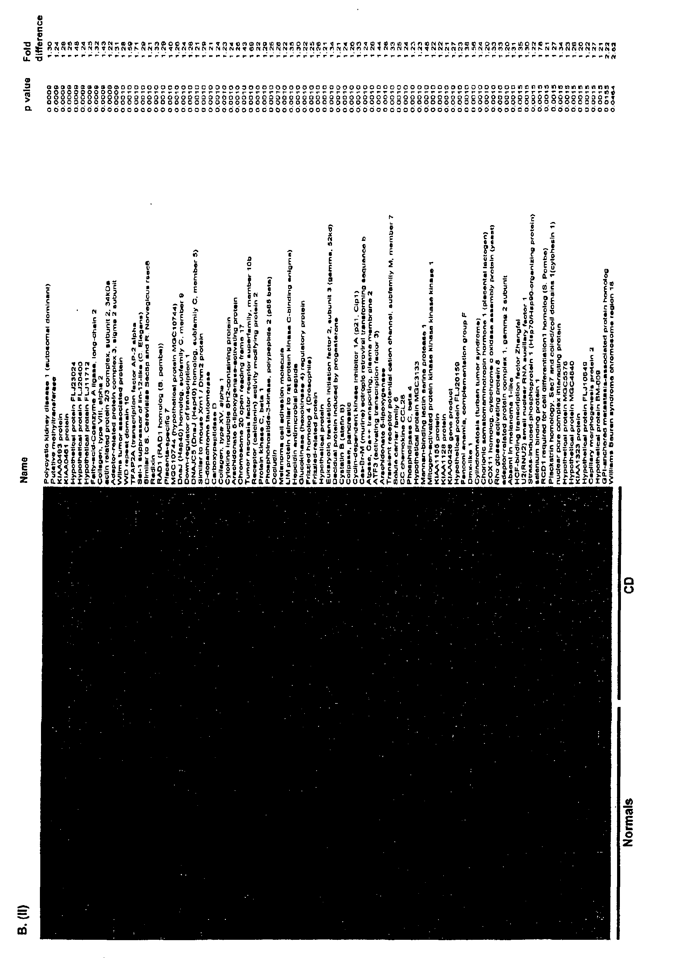

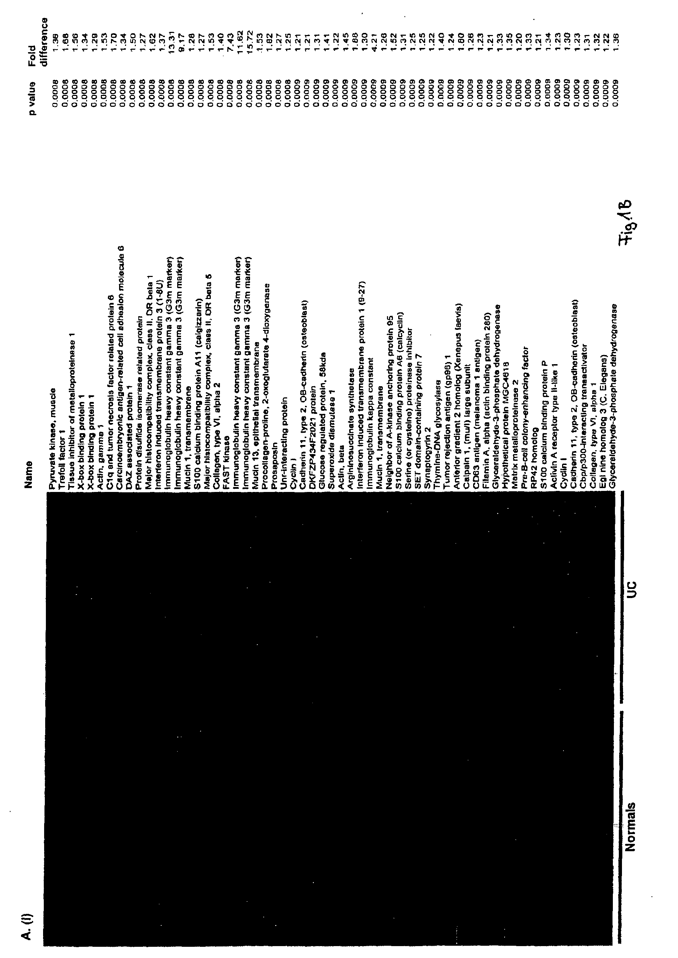

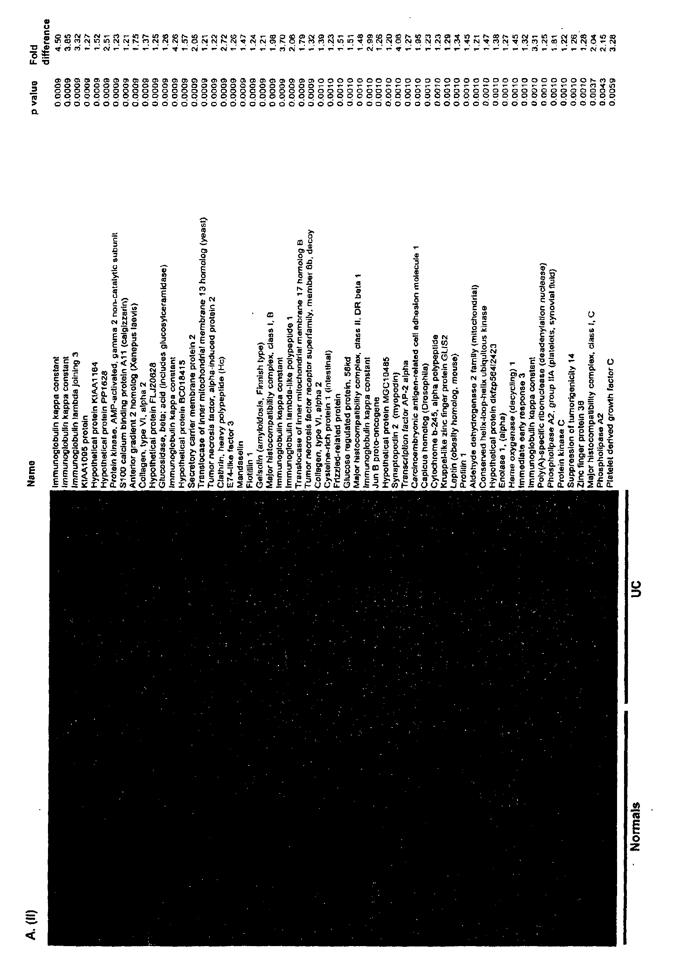

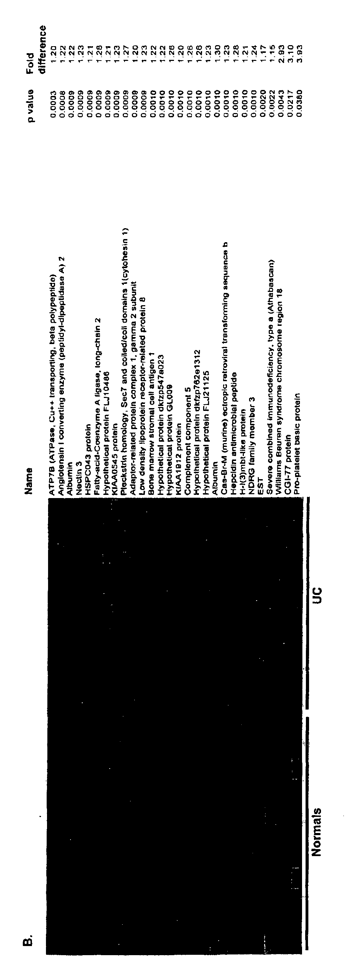

Accordingly, the present invention relates to a diagnostic composition comprising:
(i)(a) at least one nucleic acid molecule which each comprises the nucleotide sequence of a gene listed in Figure 1a or Table 4 or a fragment thereof and/or (b) at least one nucleic acid molecule which each comprises the nucleotide sequence of a gene listed in Figure 1b or Table 5 or a fragment thereof; or
(ii) at least one nucleic acid molecule which is capable of hybridizing to a nucleotide sequence of (i).

As used herein, the term "nucleotide sequence of a gene" means the sequence of the coding strand as well as the sequence of the non-coding strand.

As used herein, the term "capable of hybridizing" has the meaning of hybridization under conventional hybridization conditions, preferably under stringent conditions as described, for example, in Sambrook et al., Molecular Cloning, A Laboratory Manual, 2^{nd} edition (1989) Cold Spring Harbor Laboratory Press, Cold Spring Harbor, NY, or, preferably, as described in Example 1©, below. Also contemplated are nucleic acid molecules that hybridize at lower stringency hybridization conditions. Changes in the stringency of hybridization and signal detection are primarily accomplished through the manipulation of formamide concentration (lower percentages of formamide result in lowered stringency), salt conditions, or temperature. For example, lower stringency conditions include an overnight incubation at 37°C in a solution comprising 6X SSPE (20X SSPE = 3M NaCl; 0.2M NaH₂PO₄; 0.02M EDTA, pH 7.4), 0.5% SDS, 30% formamide, 100 µg/ml salmon sperm blocking DNA, followed by washes at 50°C with 1 X SSPE, 0.1% SDS. In addition, to achieve even lower stringency, washes performed following stringent hybridization can be done at higher salt concentrations (e.g. 5X SSC). Variations in the above conditions may be accomplished through the inclusion and/or substitution of alternate blocking reagents used to suppress background in hybridization experiments. The inclusion of specific blocking reagents may require modification of the hybridization conditions described above, due to problems with compatibility.

As a hybridization probe (or primer) nucleic acid molecules can be used, for example, that have exactly or basically the nucleotide sequence of the genes depicted in Figure 1a/1b or

Tables 4 and 5 or parts of these sequences (fragments). The term "fragment" as used herein is understood to be a part of the nucleic acid molecule that is long enough to specifically hybridize to genes of the invention or transcripts of these genes. These fragments can be used, for example, as probes or primers in a diagnostic assay. Preferably, the fragments have a length of at least 10, in particular of at least 16 and, particularly preferred, of at least 25 or at least 50 nucleotides. The nucleic acid molecules of the invention can also be used, for example, as primers for a PCR reaction. The fragments used as hybridization probe or primer can be synthetic fragments that were produced by means of conventional synthesis methods.

In a preferred embodiment, the diagnostic composition of the present invention comprises
(i)(a) at least 50 nucleic acid molecules which each comprises the nucleotide sequence of a gene listed in Figure 1a or Table 4 or a fragment thereof and/or (b) at least 50 nucleic acid molecules which each comprises the nucleotide sequence of a gene listed in Figure 1b or Table 5 or a fragment thereof; or
(ii) at least 50 nucleic acid molecules which are capable of hybridizing to 50 different nucleotide sequences of (i).

In a more preferred embodiment, the diagnostic composition of the present invention comprises
(i)(a) at least 100 nucleic acid molecules which each comprises the nucleotide sequence of a gene listed in Figure 1a or Table 4 or a fragment thereof and/or (b) at least 100 nucleic acid molecules which each comprises the nucleotide sequence of a gene listed in Figure 1b or Table 5 or a fragment thereof; or
(ii) at least 100 nucleic acid molecules which are capable of hybridizing to 100 different nucleotide sequences of (i).

In an even more preferred embodiment, the diagnostic composition of the present invention comprises
(i)(a) at least 200 nucleic acid molecules which each comprises the nucleotide sequence of a gene listed in Figure 1a or Table 4 or a fragment thereof and/or (b) at least 200 nucleic acid molecules which each comprises the nucleotide sequence of a gene listed in Figure 1b or Table 5 or a fragment thereof; or
(ii) at least 200 nucleic acid molecules which are capable of hybridizing to 200 different nucleotide sequences of (i).

In a particular preferred embodiment, the diagnostic composition comprises nucleic acid molecules comprising the nucleotide sequences of those genes listed in Figure 1a/1b and/or Table 4/5 showing the highest degree of down- or upregulation in CD or UD patients compared to normal patients.

In a further preferred embodiment, the nucleic acid molecules (or fragments) of the diagnostic composition of the present invention are bound to a solid support, for example, a polystyrene microtiter dish or nitrocellulose paper.

In an even more preferred embodiment, the nucleic acid molecules (or fragments) of the diagnostic composition are present in a microarray format which can be established according to well known methods. For example, the microarray can be produced starting from cDNA clones, plasmids etc. by PCR amplification of a given gene or gene fragment. These fragments are then spotted or robotically arrayed onto a solid support which can be, e.g., glass, nylon or any other suitable surface.

The present invention also relates to a method for diagnosing inflammatory bowel disease or a related disease, preferably Chrohn's disease (CD) or ulcerative colitis (UC) or a disposition to such a disease comprising contacting a target sample with (a) nucleic molecule(s) or fragments thereof as described above and comparing the concentration of individual mRNA(s) with the concentration of the corresponding mRNA(s) from at least one healthy donor. An aberrant (increased or decreased) mRNA level of at least one gene, preferably at least 50 genes, more preferably at least 100 genes and, even more preferably at least 200 genes determined in the sample in comparison to the control sample is an indication of inflammatory disease or a related disease or a disposition to such kinds of diseases. For diagnosis, samples are, preferably, obtained from inflamed colon tissue. For analysis of gene expression, total RNA is obtained according to standard procedures and, preferably, reverse-transcribed, e.g. as described in Example 1, below. Preferably, a DNAse treatment (in order to get rid of contaminating genomic DNA) is performed.

The nucleic acid molecule or fragment is typically a nucleic acid probe for hybridization or a primer for PCR. The person skilled in the art is in a position to design suitable nucleic acids probes based on the information provided in the Figures and Tables of the present invention. The target cellular component, i.e. mRNA, e.g., in colon tissue, may be detected directly in situ, e.g. by in situ hybridization or it may be isolated from other cell components by common methods known to those skilled in the art before contacting with a probe. Detection methods include Northern blot analysis, RNase protection, in situ methods, e.g. in situ hybridization, in vitro amplification methods (PCR, LCR, QRNA replicase or RNA-transcription/amplification (TAS, 3SR), reverse dot blot disclosed in EP-B1 0 237 362)) and other detection assays that are known to those skilled in the art. Products obtained by in vitro amplification can be detected according to established methods, e.g. by separating the products on agarose gels and by subsequent staining with ethidium bromide. Alternatively, the amplified products can be detected by using labeled primers for amplification or labeled dNTPs. Preferably, detection is based on a microarray.

The probes (or primers) (or, alternatively, the reverse-transcribed sample mRNAs) can be detectably labeled, for example, with a radioisotope, a bioluminescent compound, a chemiluminescent compound, a fluorescent compound, a metal chelate, or an enzyme.

The present invention also relates to the use of the nucleic acid molecules or fragments described above for the preparation of a diagnostic composition for the diagnosis of inflammatory bowel disease or a related disease or a disposition to such a disease.

Finally, the present invention relates to the use of the nucleic acid molecules of the present invention for the isolation or development of a compound which is useful for therapy of inflammatory bowel disease, e.g., Chrohn's disease (CD) or ulcerative colitis (UC). For example, the nucleic acid molecules of the invention and the data obtained using said nucleic acid molecules for diagnosis of inflammatory bowel disease might allow to identify further genes which are specifically dysregulated, thus may be considered as potential targets for therapeutic interventions.

### Brief description of the drawings

Figure 1a: Heat Map of differentially expressed annotated genes between normal (n=11) and CD (n=10)
   Individual genes (sorted by p-value) are in rows and individual filters (or experiments) are in columns. Varying levels of expression are represented on a scale from green (low expression), black (intermediate expression) and bright red (high expression). Out of a 258 annotated genes, panel A shows up-regulated (81 genes) and panel B shows down-regulated genes (177 genes).
Figure 1b: Heat Map of differentially expressed annotated genes between normal (n=11) and UC (n=10)
   Individual genes (sorted by p-value) are in rows and individual filters (or experiments) are in columns. Varying levels of expression are represented on a scale from green (low expression), black (intermediate expression) and bright red (high expression). Out of a 145 annotated genes, panel A shows up-regulated (117 genes) and panel B shows down-regulated genes (28 genes).
Figure 2A: Real-time TaqMan results between normal and CD patient populations
   Statistically significant differences between control and CD samples were determined using an Anova single factor test followed by the appropriate post-hoc test (student t-test). Bars indicate mean values. * = p < 0.05; **p < 0.01. n=11 normals and n=10 CD unless otherwise indicated (in brackets).
Figure 2B: Real-time TaqMan results between normal and UC patient populations
   Statistically significant differences between control and UC samples were determined using an Anova single factor test followed by the appropriate post-hoc test (student t-test). Bars indicate mean values. * = p < 0.05; **p < 0.01. n=11 normals and n=10 UC unless otherwise indicated (in brackets).
Figure 3: Section of the Unigene filter prior to hybridization
   The inherent fluorescence observed when spotting on nylon membranes was used to determine the quality of filter spotting. 1.58 mM fluorescin was added to the *Arabidopsis Thaliana* control plate (two bright spots) and the filter was scanned using an in-house work station for high resolution fluorescence detection (MPI for Molecular Genetics, Berlin). The Unigene PCR plates had no fluorescence marker added but could be clearly visualised (fainter spots), as were the four blank positions in each 6 X 6 spotting pattern. In this way, unevenly or poorly spotted filters were easily identified and discarded.

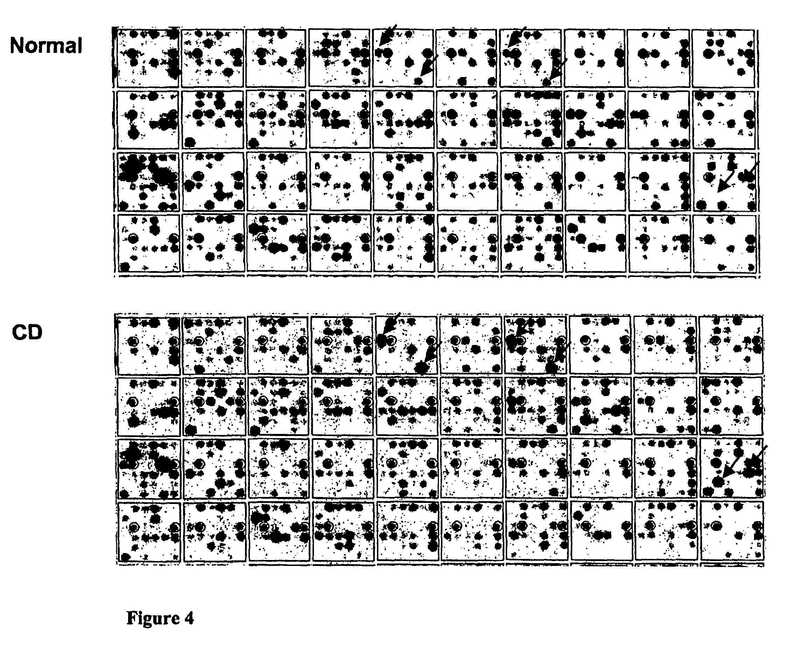

Figure 4: Sub-section of gridded Unigene filters hybridised with radiolabelled cDNA
   Panel A represents radiolabelled cDNA from the non-inflamed region of a control patient whereas Panel B represents hybridisation with a highly inflamed CD sigma sample. Example duplicate clones in these sections of the Unigene filter that show differential expression patterns between the two conditions are indicated by arrows.

The below examples explain the invention in more detail.

### Example 1

### General Methods

### (A) Study Design

Eleven individuals (5F; mean age: 51.4 yrs (range: 25-84 yrs)) were included in this study as the normal population, as subsequent endoscopic and histological examination yielded no significant pathological findings in this group. Indications for colonoscopy included cancer screening and previous intermittent diarrhoea. To investigate gene expression in IBD, ten CD (4F; mean age: 33.6 yrs (range: 19-66 yrs)) and ten UC (2F; mean age: 34.8 yrs (range: 27-51 yrs)) patients we recruited. To reduce expression variability due to taking biopsies from different regions of the colon, all endoscopic biopsies were taken from the sigmoid colon, and immediately snap-frozen in liquid nitrogen. In order to qualify for inclusion into the study, IBD patients had to be endoscopically clinically active at the time of sampling but free of all medication for a minimum of 6 weeks prior to endoscopy. Histopathological analysis including disease activity scoring was carried out by a single pathologist blinded to both disease status and the expression screening results. The normal group showed no pathologic signs of activity (data not shown) whereas the diseased population represented a broad range of both active and chronic inflammation (Table 1). Patients consented to the additional research biopsies being taken 24 hrs prior to endoscopy, and the study procedures were approved by the hospital ethical committee.

**Table 1**

| **Clinical characteristics and histopathological findings of the IBD patient population** | | | | | | | | | | | | | | | | | | | | |
|---|---|---|---|---|---|---|---|---|---|---|---|---|---|---|---|---|---|---|---|---|
| | ***Crohn's* Disease** | | | | | | | | | | **Ulcerative Colitis** | | | | | | | | | |
| | P1 | P2 | P3 | P4 | P5 | P6 | P7 | P8 | P9 | P10 | P1 | P2 | P3 | P4 | PS | P6 | P7 | P8 | P9 | P10 |
| Age (yr) Sex Activity Index Chronicity Index Additional Microscopic findings | 20 | 38 | 19 | 32 | 24 | 66 | 25 | 44 | 50 | 18 | 29 | 38 | 27 | 31 | 29 | 38 | 33 | 51 | 29 | 43 |
| | M | F | M | F | F | M | M | M | M | F | M | M | M | M | M | F | M | M | F | M |
| | A0 | A2 | A3 | A1 | A0 | A1 | A1 | A1 | A2 | A2 | A3 | A1 | A1 | A0 | A1 | A2 | A2 | A3 | A1 | A3 |
| | C2 | C2 | C3 | C3 | C1 | C1 | C1 | C2 | C2 | C2 | C3 | C3 | C2 | C1 | C2 | C3 | C2 | C3 | C3 | C3 |
| | E1, | L | E1, | E1, | E1 | L | | E1, | L | P | L | E1, | E2, | | P | | | | P | |
| | L | | E2 | E2 | | | | L | | | | E2, L | P | | | | | | | |
| Activity Index: A0: No active crypt injury, A1: Focal cryptitis or crypt abscess, no ulceration; A2: Disseminated cryptitis & crypt abscesses, no ulceration; A3: Ulceration; Chronicity Index: C0: No chronic mucosal injury; C1: Increased lymphoplasmocytic infiltration, C2: Mucosal and submucosal fibrosis; C3: Mucosal atrophy; Additional microscopic findings: E1: Eosinopholic rich infiltrate; E2: Eosinophilic cryptitis, P: Paneth cell metaplasia, L: Lymphoid aggregates with germinal centres. All samples were taken form the sigmoid colon. | | | | | | | | | | | | | | | | | | | | |

### (B) Preparation of high density cDNA arrays

cDNA clone inserts from eighty-eight 384-well microtiter plates (33,792) from the Human UniGene set RZPD 1 (Resource Centre and Primary Database GmbH, Berlin, Germany) clone set (http://www.rzpd.de - Build 17, NCBI) were amplified by PCR using M13 forward (5'-CGTTGTAAAACGACGGCCAGT-3')and reverse (5-TTTCACACAGGAAACAGCTAT GAC-3') primers. Since the redundancy of this clone set was previously estimated to be 1.44-fold, the 33,792 clones on the cDNA array represent approximately 23,466 unique transcripts. The PCR reaction was carried out in a final volume of 25 µl containing 1 X PCR buffer (0.5 M Tris-HCl (pH 8.6), 0.5 M KCl, 15 mM MgCl₂, 1% Tween 20), betaine (1.5 U), 0.2 mM dNTPs, 0.2 µM primers and 0.1 U/µl Taq/Pfu (Promega, Madison, WI, USA). Amplification of PCR products was carried out as follows: 95ºC for 1 min, 94ºC for 15 sec, 59ºC for 15 sec, 65ºC for 55 sec, repeat of 59ºC for 15 sec, 65ºC for 55 sec for an additional 2 cycles, repeat of 94ºC for 15 sec, 59ºC for 15 sec, 65ºC for 55 sec for 29 cycles and finally 68ºC for 10 min. The sub-cycling step described improved product yield and stability. The quality of the PCR reaction was determined visually by slot-blot electrophoresis on 1% agarose gels.

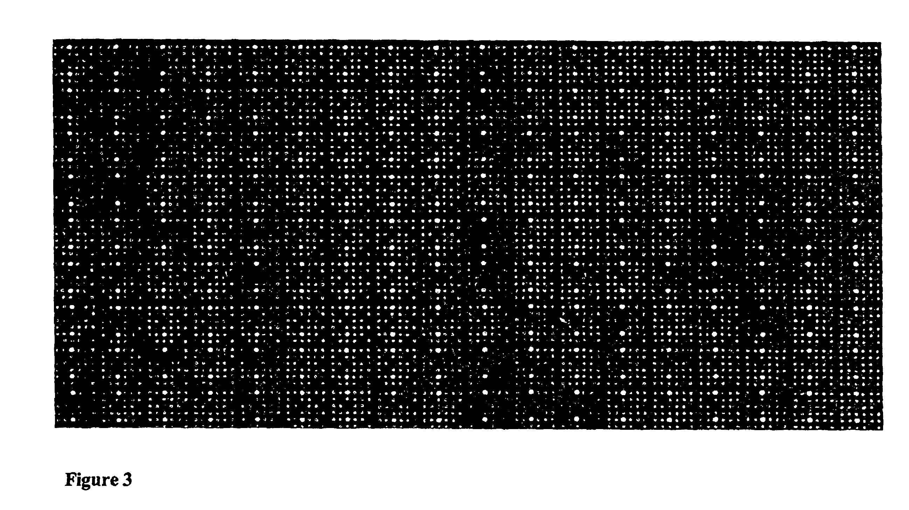

Prior to spotting, PCR products were denatured by the addition of a final concentration of 0.1 M NaOH to each well. The 33,792 PCR products were spotted in duplicate in a 6 X 6 pattern on dry 23 X 23 cm Hybond N⁺ nylon membranes (Amersham Pharmacia, Freiburg, Germany) using a Genetix robot (New Milton, Hampshire, UK) attached to a 384 pin gadget-head (250 µm pins). Duplicate *Arabidoposis thaliana* clones (GenBank accession number: U29785) served as guide spots for gridding purposes. In order to determine the quality of the spotting, 1.58 µM fluorescin was added to the *Arabidoposis thaliana* control clone plate and the filter was scanned using a self-constructed work station for high resolution fluorescence detection (MPI for Molecular Genetics, Berlin - see Figure 3). In this way, unevenly or poorly spotted filters were discarded prior to hybridisation. After spotting, cDNA filters were treated as previously described (Boer et al., Genome Res. 11 (2001), 1861-1870) and stored dry at 4°C until ready for use.

### (C) Target Preparation and Hybridisation Procedure

Snap-frozen biopsies were crushed to a fine powder under liquid nitrogen using a manual crusher with a teflon head (Omnilab, Bremen, Germany) and total RNA was isolated using the RNeasy Mini Kit (Qiagen, Hilden, Germany) according to the manufacturers protocol, with the exception that DNase treatment was carried out X 3. Eluted RNA was determined to be intact as assessed by standard formaldehyde gel electrophoresis and shown to be free of contaminating genomic DNA when primers specific for GAPDH yielded a PCR product only after reverse transcription. Typically, 0.8-1.2 µg of total RNA per mg of tissue were obtained with this method.

200-500 ng mRNA were isolated from total RNA for each patient using the Oligotex kit (Qiagen). Prior to hybridisation, 20 ng of *Arabidopsis thaliana* DNA template was labelled with α-³³P dCTP (Feinberg and Vogelstein, Anal. Biochem. 132 (1983), 6-13). Reversed transcribed cDNAs from patient targets were prepared in a final volume of 30 µl as previously described (Eickhoff et al., Genome Res. 10 (2000), 1230-1240), with the exception that the labelling reaction was carried out at 42°C for 50 min. RNA was hydrolysed with the addition of 3 M NaOH (68°C for 20 min), neutralised and unincorporated radioactive nucleotides removed (Microspin G-50 columns, Amersham Pharmacia). Prior to each hybridisation reaction, unused filters were initially washed in 1 X SSC; 0.1% SDS at RT for 10 min twice followed by three washes in 0.1 X SSC; 0.1% SDS for 20 min at 80ºC. Filter was pre-hybridised for 2 hrs at 50ºC in the following hybridisation solution; 7% SDS, 50% formamide, 5 X SSC, 2% blocking reagent (Roche Diagnostics, Basel, Switzerland), 50mM sodium phosphate (pH 7.0) and denatured herring sperm DNA (50 µg/ µl). Following hybridisation (overnight at 42ºC), membranes were washed in 1 X SSC/0.1% SDS buffer for 10 min at RT followed by 2 washes for 30 min in 0.2 X SSC/0.1% SDS at 65ºC. Filters were exposed to imaging plates for 20 hrs (BAS-MS 2325) and scanned at 50 µm resolution on FLA-3000G phosphoimager (Fuji-film Medical Systems, Stanford, CT, USA). A typical hybridisation image is shown in Figure 5.

### (D) Data Analysis

Image gridding was carried out using VisualGrid® (GPC Biotech, Martinsried, Germany) software and results were imported into a custom-made database. The ranked expression data distribution was found to follow Zipf's law and the log normal transformed datasets were normalised following this principle (Hoyle et al., Bioinformatics 18 (2002), 576-584). Any hybridisation signals that had a value that fell below +2SD of the mean background were considered as indistinguishable from background and was set to zero, and those few values where duplicate expression values for the same genes were not similar (outliers or false positives) were eliminated. The entire microarray data-set was not normally distributed and therefore, in order not to assume a specific distribution of data, significantly differentially regulated genes between conditions were identified by a non-parametric test (Mann-Whitney U). A recently published comparison of microarray data analysis techniques showed low false positive rates with non-parametric analysis if a low p-value cut-off was applied (Troyanskaya et al., Bioinformatics 18 (2002), 1454-1461). Therefore, in order to minimise false positives, a stringent p-value < 0.001 was chosen as significant (> 1.2 fold change). Any transcript that had a fold difference > 2.0, but did not meet the p-value cut-off was also included in the final table, as the few genes that fell into this category were biologically relevant to disease.

Heat-maps of differentially regulated genes that were annotated were generated using "Spotfire" software (Sornerville, MA, USA).

### (E) Verification of clone sequences

Differentially expressed clones were sequenced from bacterial stock plates. Bacteria were allowed to grow in LB medium (with 100 µg/ml of Ampicillin) for 20-24 hrs at 37ºC. Plasmid isolation was carried out using the Montage Plasmid Miniprep₉₆ kit (Millipore, Eschborn, Germany). The sequencing reaction was carried out in a final volume of 10 µl, using 1 µl (10 pmol/µl) of either the forward (5' GACGTTGTAAAACGACGGCCAGT-3') or reverse (5'-ATAACAATTTCACACAGGAAACAGCTATGA-3') primer, 3 µl Big Dye Terminator, 1 µl of water and 5 µl of plasmid prep (-80 ng/ml) and run on the ABI-3700 capillary sequencer (Applied Biosystems, USA). Contigs were generated if the clone ends overlapped (Sequencher software, Gene Codes Corporation, Ann Arbor, MI, USA) and identified by BLAST searching (www.ncbi.nlm.nih.gov/BLAST/). Only sequence verified ESTs are presented.

### (F) Verification of hybridisation results

Selected gene expression signals were independently verified with real-time quantitative PCR (TaqMan, Applied Biosystems Corp., Foster City, CA, USA). Verified sequences of the corresponding clones were scanned for repeat regions (http://repeatmasker.genome.washington.edu/cgi-in/RepeatMasker). Probe and primers were designed using Primer Express software, version 2.0 (Applied Biosystems) (see Table 2). Prior to real-time analysis, a conventional PCR reaction with control cDNA (isolated from pooled intestinal surgical samples) was carried out with each primer pair to ensure that only the expected product amplicon size was obtained.

Total RNA (1 µg), from the same biopsies used for the hybridisation experiments, was reverse-transcribed to cDNA with the TaqMan reverse transcription kit (PE Applied Biosystems). Real time TaqMan PCR was performed using β-actin as the endogenous control gene according to the TaqMan PCR master-mix protocol (Applied Biosystems). Probes and primers were designed in-house with the exception of the house keeping gene (Kreuzer et al., Clin. Chem. 45 (1999), 297-300) (see Table 2). Reactions were carried out on the ABI PRISM 7700 Sequence Detection System (PE Applied Biosystems) and mRNA levels were determined using the comparative Ct method (PE Applied Biosystems, User Bulletin #2, ABI Prism 7700 SDS, 1997). Statistically significant differences between control and IBD samples were determined using an one-way analysis of variance (ANOVA) followed by the appropriate post-hoc test (two-tailed student t-test assuming equal or unequal variances). A p-value of < 0.05 was considered significant.

### (G) Functional prediction of unknown ESTs

In order to identify novel genes, unknown differentially regulated clones were examined in more detail. cDNA clone sequences were searched against the GenBank non-redundant database by means of BLAST searching. The best BLAST hit (sequence identity close to 100%) was used to extend the sequences to longer mRNA transcripts. InterPro (http://www.ebi.ac.uk/interpro/scan.html), SMART (http://smart.embl-heidelberg.de) and Pfam (http://www.sanger.ac.uk/Software/Pfam) search methods (with standard parameter sets) were used to detect known protein domains and functional sequence motifs in putative open reading frames (ORFs) longer than 100 amino acids. Close homologies of the ORFs were found through their association to UniGene clusters in humans and other species. In cases of ORFs without a detectable homology to known genes, these transcripts were mapped to genomic location by BLAST searching and examined in terms of their genomic context.

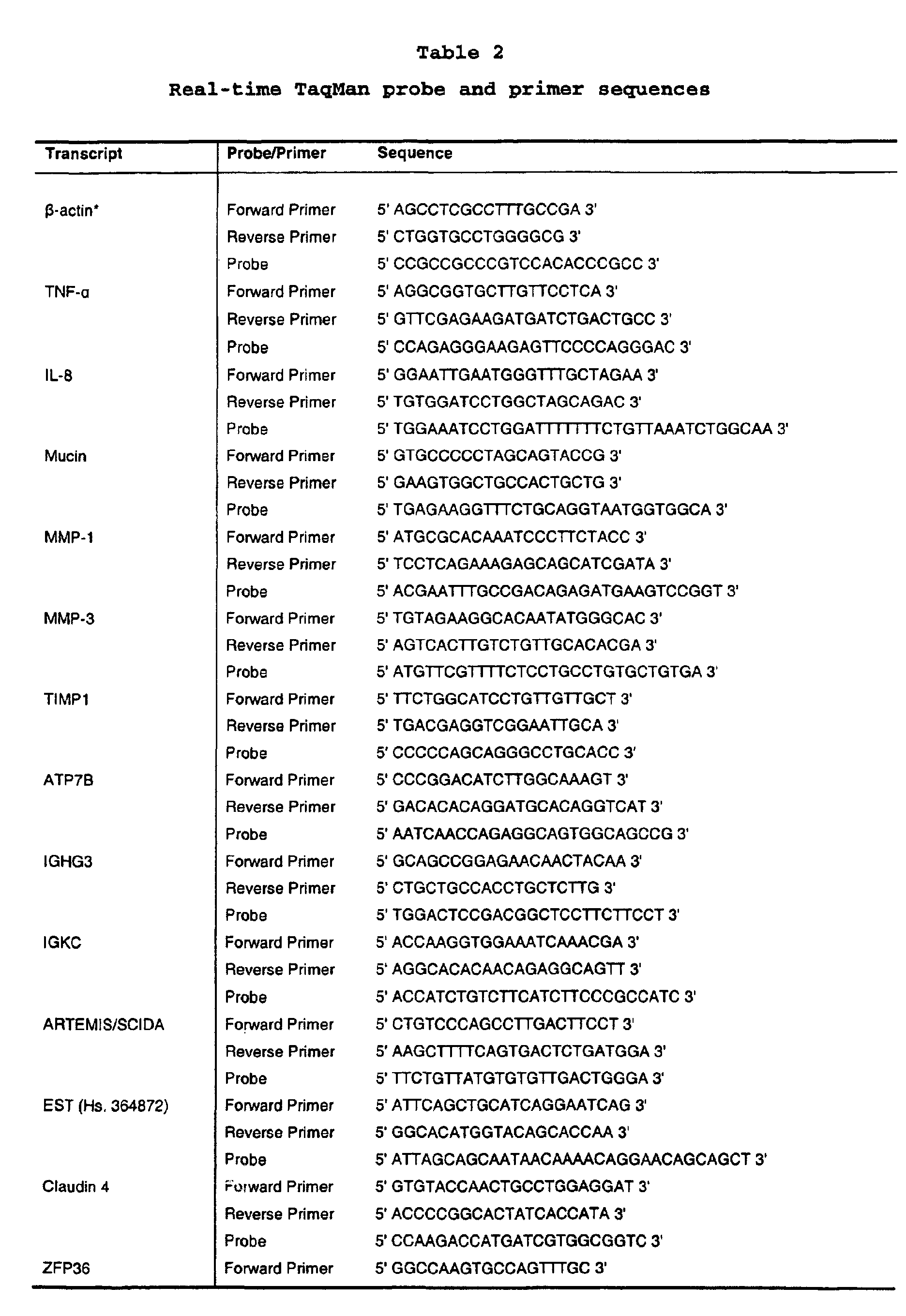

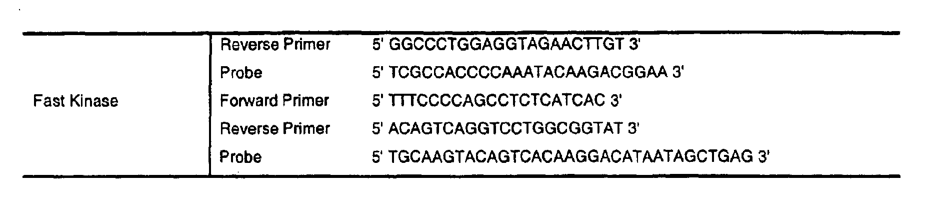

All probe and primers were made to order by Eurogentec (Seraing, Belgium) and were used at final concentrations of 200nM and 300nM respectively. *β-actin primers were previously published by Kreuzer et al., Clin. Chem. 45 (1999), 297-300. Probes were labelled with a reporter dye, 6-carboxy-fluorescein (6-FAM), covalently attached to the 5' end, and a quencher dye, 6-carboxy-tetramethyl-rhodamine (TAMRA) at the 3' terminus of the probe sequence.

### Example 2

### Identification and characterisation of genes which are aberrantly expressed in inflammatory bowel disease

### (A) Microarray data quality

In order to ascertain data reliability and reproducibility, the expression levels of each of the 33,792 clones were measured in duplicate in one experiment. The resulting correlation coefficient was very high (r=0.99), indicating that hybridisation signals were highly reproducible (data not shown). To assess array and hybridisation-based experimental variability, hybridisation was performed on five separate arrays using the same RNA sample (extracted from an intestinal surgical specimen). The inter-assay variability for each of the 33,792 duplicate signals was calculated and using a 0.001 confidence level, a fold difference > 1.2 was defined as a significant technical threshold for comparative profiling between normal and diseased samples.

In order to determine the degree of biological diversity between individuals (inter-individual variation), 11 normal sigmoid colon samples were compared. By calculating variation coefficients for all array features, estimations of the variability for the entire filter could be calculated. Approximately 78% of transcripts had a variation coefficient of < 0.3 indicating that most genes showed little expression or had low variability. However, 20% of transcripts were highly variable in normals (variation coefficients: 0.3 - 0.5), and their expression levels were distributed over the full dynamic range of the system. Therefore, a comparative study has to define abnormalities in a disease on the basis of a comparison to gene-specific normal variability. This would indicate that pooling of samples should be avoided when carrying out microarray experiments. Approximately 2% of samples showed very high variability (> 0.5) but reflect signals at the lower limits of detection of the system.

### (B) Comparison of the normal and IBD intestine

Normal sigmoid samples (n=11) and samples from both CD (n=10) and UC (n=10) patients were used to compare differences between the normal and inflamed intestine. All differentially expressed genes reported in this study were sequence verified, and incorrect annotations were found in 27% of cases. This error rate is similar to that reported for different IMAGE clone sets in other studies. After implementing the analysis criteria, 465 differentially regulated transcripts (91 were upregulated) were identified between normal and CD patients, and 209 differentially transcripts (147 were upregulated) were identified between normal and UC patients. Of these, approximately 45% and 30% represented unknown genes in CD and UC, respectively. Some of these differentially regulated genes were located in chromosomal areas previously implicated in linkage studies (Table 3).

**Table 3**

| **Differentially regulated annotated genes in IBD linkage regions identified on arrays** | | | | | |
|---|---|---|---|---|---|
| *Name* | *Symbol* | *Chromosome*/*Cytoband* | *IBD locus* | *Direction of change* | *Disease status* |
| Cylindromatosis (turban tumor syndrome) | CYLD | 16q12.1 | IBD1 | Down | CD |
| Glycine cleavage system protein H | GCSH | 16q23.3 | IBD1 | Down | CD |
| Solute carrier family 6, member 2 | SLC6A2 | 16q12.2 | IBD1 | Down | CD |
| Cadherin 11, type 2, OB-cadherin (osteoblast) | CDH11 | 16q22.1 | IBD1 | Up | CD & UC |
| Cytochrome b-245, alpha polypeptide | CYBA | 16q24 | IBD1 | Up | CD & UC |
| Matrix metalloproteinase 2 | MMP2 | 16q13-q21 | IBD1 | Up | CD |
| 2'-5'-oligoadenylate synthetase 2 (69-71 kd) | OAS2 | 12q24.2 | IBD2 | Down | CD |
| Tachykinin 3 (nouromedin K, neurokinin beta) | TAC3 | 12q13-q21 | IBD2 | Down | CD |
| Ubiquitin specific protease 15 | USP15 | 12q14 | IBD2 | Down | CD |
| Activin A receptor type II-like 1 | ACVRL1 | 12q11-q14 | 18D2 | Up | UC |
| Aldehyde dehydrogenase 2 family (mitochondrial) | ALDH2 | 12q24.2 | IBD2 | Up | UC |
| CD63 antigen (melanoma 1 antigen) | CD63 | 12q12-q13 | IBD2 | Up | UC |
| Thymine-DNA glycosylase | TDG | 12q24.1 | IBD2 | Up | UC |
| Tumor rejection antigen (gp96) 1 | TRA1 | 12q24.2-q24.3 | IBD2 | Up | CD & UC |
| Unr-interacting protein | UNRIP | 12p13.31 | IBD2 | Up | UC |
| Cyclin-dependent kinase inhibitor 1A (p21, Cip1) | CDKN1A | 6p21.2 | IBD3 | Down | CD |
| Colipase, pancreatic | CLPS | 6pter-p21.1 | IBD3 | Down | CD |
| Major histocompatibility complex, class II, DO alpha 1 | HLA-DQA1 | 6p21.3 | IBD3 | Down | CD |
| Transcription factor 21 | TCF21 | 6pter-qter | IBD3 | Down | CD |
| Zinc finger protein 204 | ZNF204 | 6p21.3 | IBD3 | Down | CD |
| Flotillin 1 | FLOT1 | 6p21.3 | IBD3 | Up | UC |
| Major histocompatibility complex, class I, B | HLA-B | 6p21.3 | IBD3 | Up | CD & UC |
| Major histocompatibility complex, class I, C | HLA-C | 6p21.3 | IBD3 | Up | UC |
| Major histocompatibility complex, class II, DR beta 1 | HLA-DRB1 | 6p21.3 | IBD3 | Up | UC |
| Major histocompatibility complex, class II, DR beta 3 | HLA-DR83 | 6p21.3 | IBD3 | Up | CD |
| Major histocompatibility complex, class II, DR beta 5 | HLA-DRB5 | 6p21.3 | IBD3 | Up | UC |
| Immediate early response 3 | IER3 | 6p21.3 | IBD3 | Up | CD & UC |
| Adaptor-related protein complex 1, gamma 2 subunit | AP1G2 | 14q11.2 | IBD4 | Down | CD & UC |
| Leukotriene B4 receptor BLT2 | BLTR2 | 14q11.2-q12 | IBD4 | Down | CD |
| DAZ associated protein 1 | DAZAP1 | 19p13.3 | IBD6 | Up | UC |
| Jun B proto-oncogene | JUNB | 19p13.2 | IBD6 | Up | CD & UC |
| Lamin B2 | LMNB2 | 19p13.3 | IBD6 | Up | CD |
| Neighbor of A-kinase anchoring protein 95 | NAKAP95 | 19p13.13 | IBD6 | Up | UC |
| Poly(A) binding protein, cytoplasmic 4 (inducible form) | PABPC4 | 1p32-p36 | IBD7 | Down | CD |
| Tumor necrosis factor receptor superfamily, member 12 | TNFRSF12 | 1 p36.2 | IBD7 | Down | CD |
| Enolase 1, (alpha) | ENO1 | 1p36.3-p36.2 | IBD7 | Up | CD & UC |
| Kinesin family member 18 | KIF18 | 1p36.2 | IBD7 | Up | CD |
| Nuclear pore complex interacting protein | NPIP | 16p13-p11 | IBD8 | Down | CD |
| Polycystic kidney disease 1 (autosomal dominant) | PKD1 | 16p13.3 | IBD8 | Down | CD |
| Protein kinase C, beta 1 | PRKCB1 | 16p11.2 | IBD8 | Down | CD |
| Kruppel-like zinc finger protein GLIS2 | GLIS2 | 16p13.3 | IBD8 | Up | UC |
| Poly(A)-specific ribonuclease (deadenylation nuclease) | PARN | 16p13 | IBD8 | Up | UC |
| IBD locus is the classification according to OMIM and only those genes in IBD1-IBD8 are listed in this table. For a complete listing of genes, in other IBD loci available in the literature, see supplemental tables B & C. | | | | | |

An interesting finding was that only 93 genes were common to both diseases, suggesting that these disease subtypes are substantially different. Transcripts identified reflected broad biological processes such as pathogenesis (HLA-DQA1 (Hs.198253) & HLA-DRB1 (Hs.308026)), cell growth and/or maintenance (occludin (Hs.171952), and kinases such as MAP4K1 (Hs.86575) and PRKCB1 (Hs.77202)), developmental processes (collagen (Hs.159263) and CHUK (Hs. 198998)), physiological processes (claudin 4 (Hs.5372)), cell adhesion (cadherin 11 (Hs.75929)), and oncogenesis (AGR2 (Hs.91011) & CDKN1A (Hs.179665)). The largest category was that of immunity/defence which contain such proteins as XBP1 (Hs.149923), IL1R1 & 2 (Hs.82112 & Hs.25333) and IGKC (Hs.156110), to name but a few. A more comprehensive list of all differentially regulated genes (annotated and unannotated) can be found in Tables 4 and 5, while all annotated genes are presented in Figures 1a and 1b. Table 4 and 5 show differentially regulated genes between normal and UC patients (Table 4) and CD patients (Table 5), respectively, in 21 separate assays. The Accession numbers, cytoband location (where known), average expression values, permutated p-values, fold change and the direction of change are shown (+, up-regulated; -, down-regulated). Data is sorted by descending fold change.

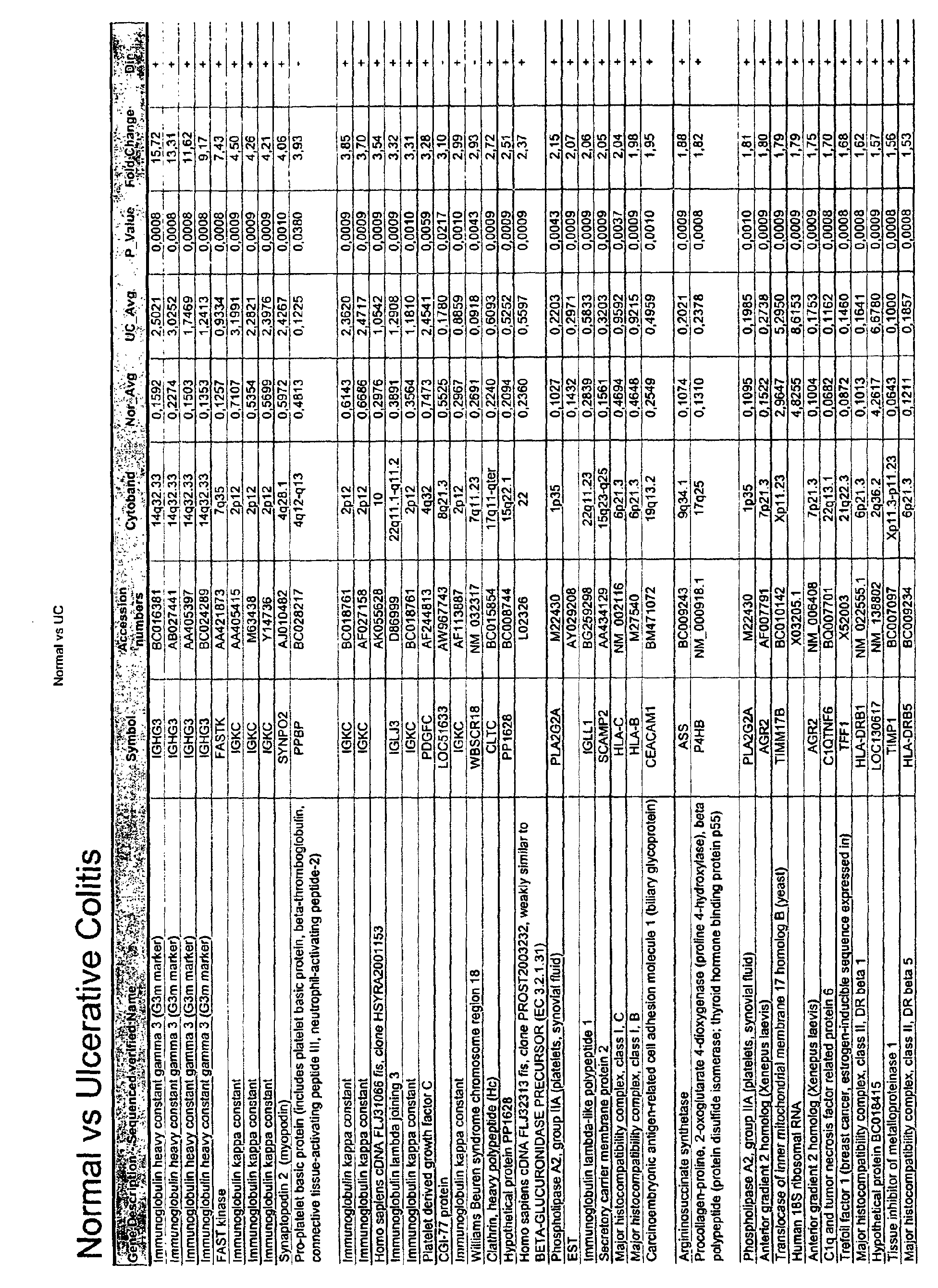

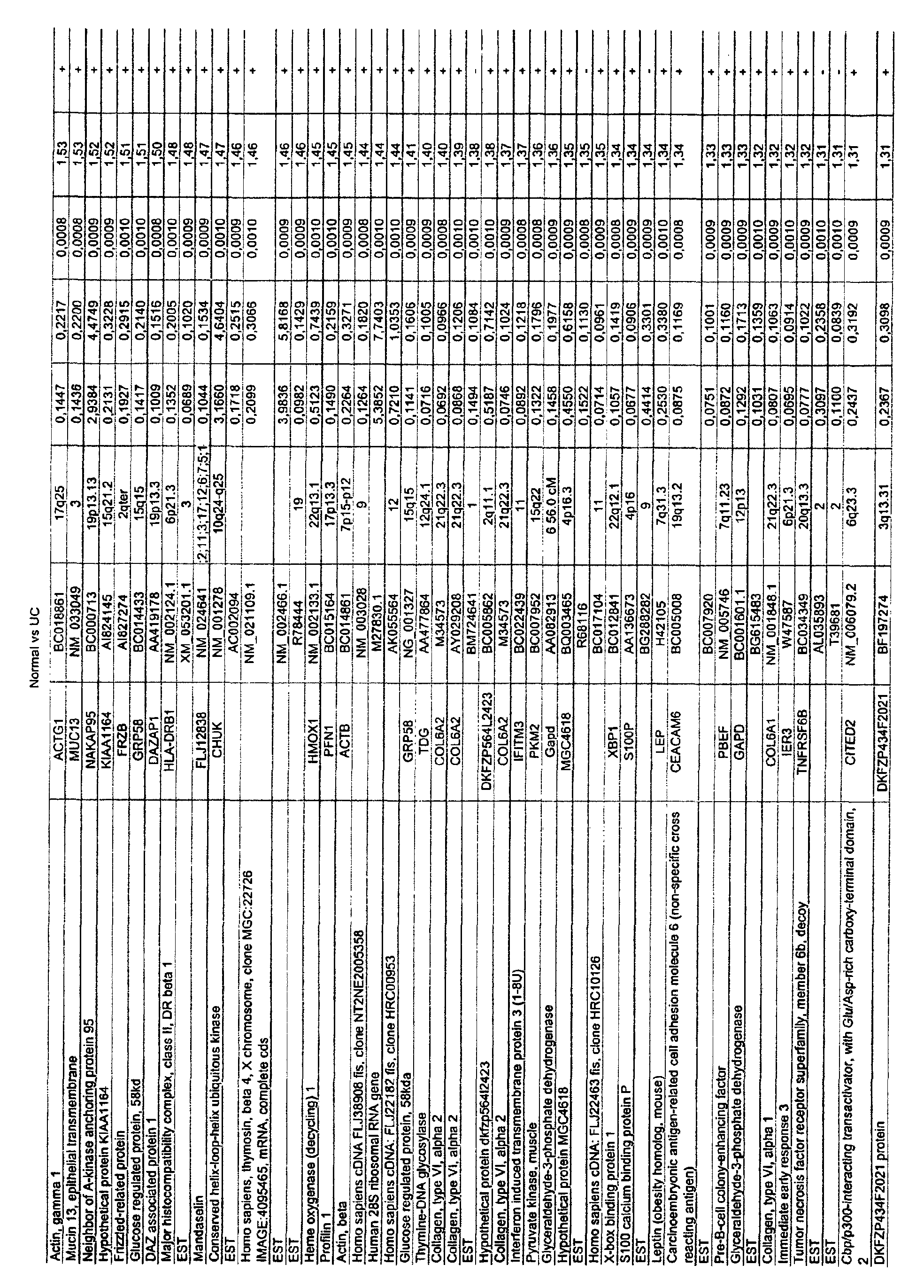

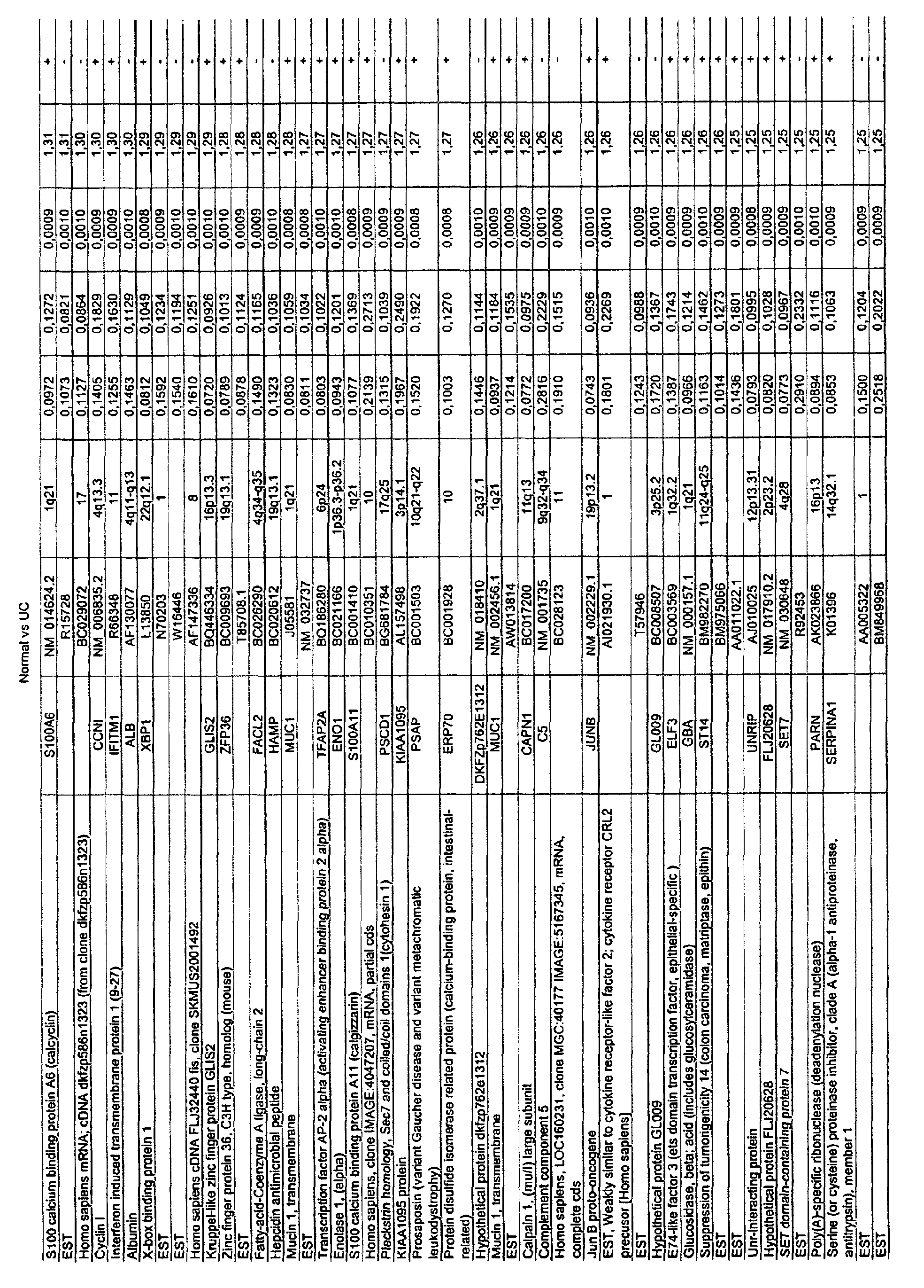

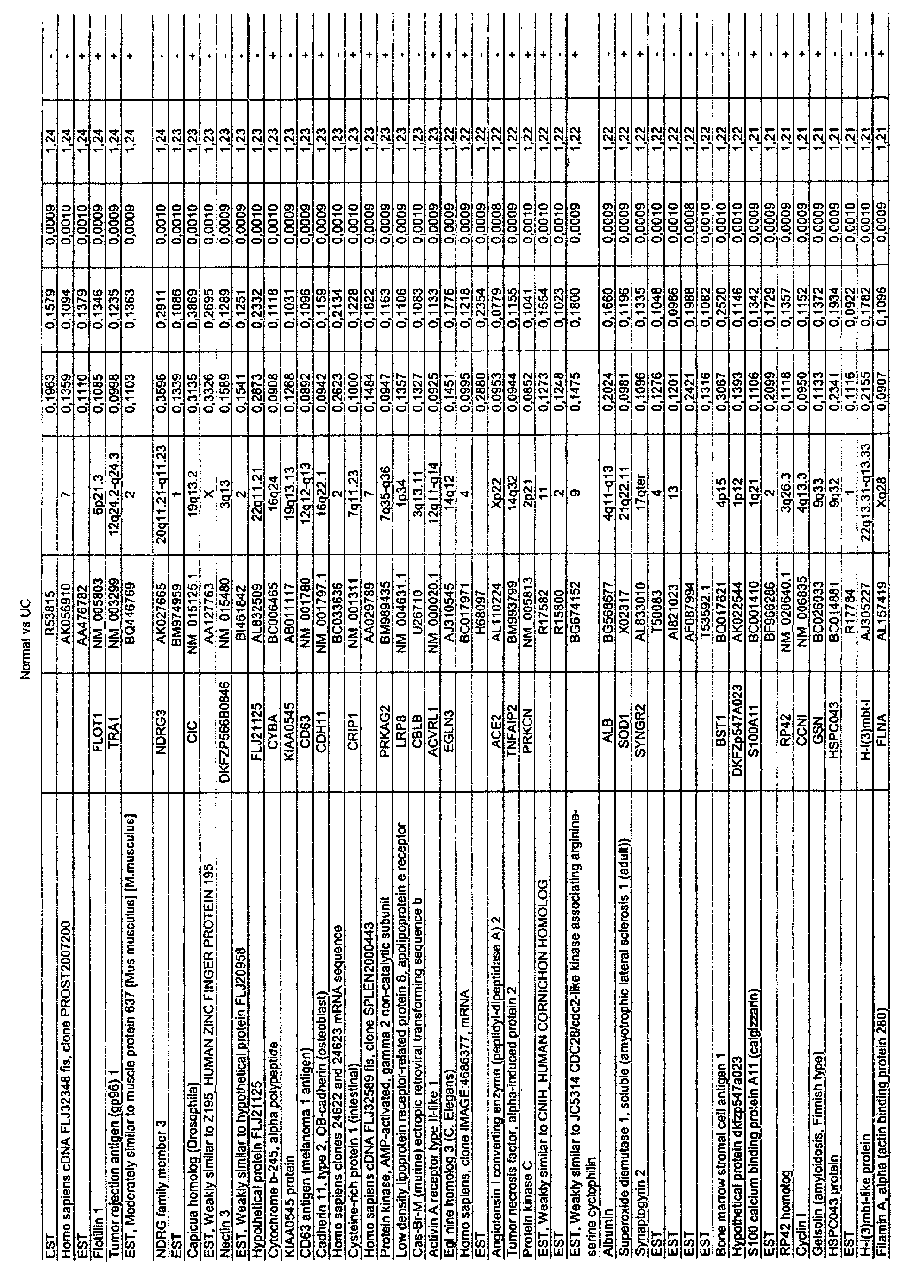

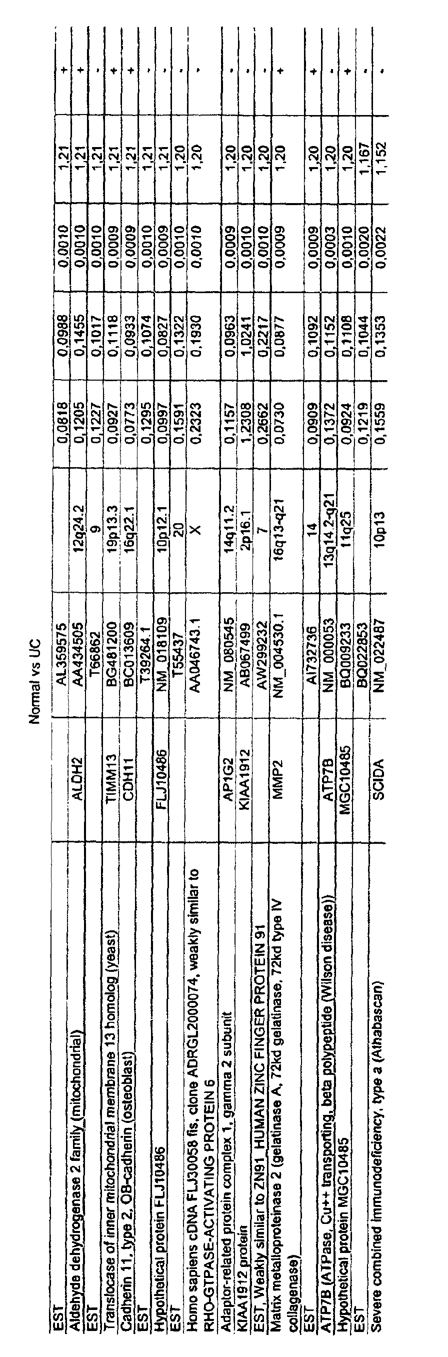

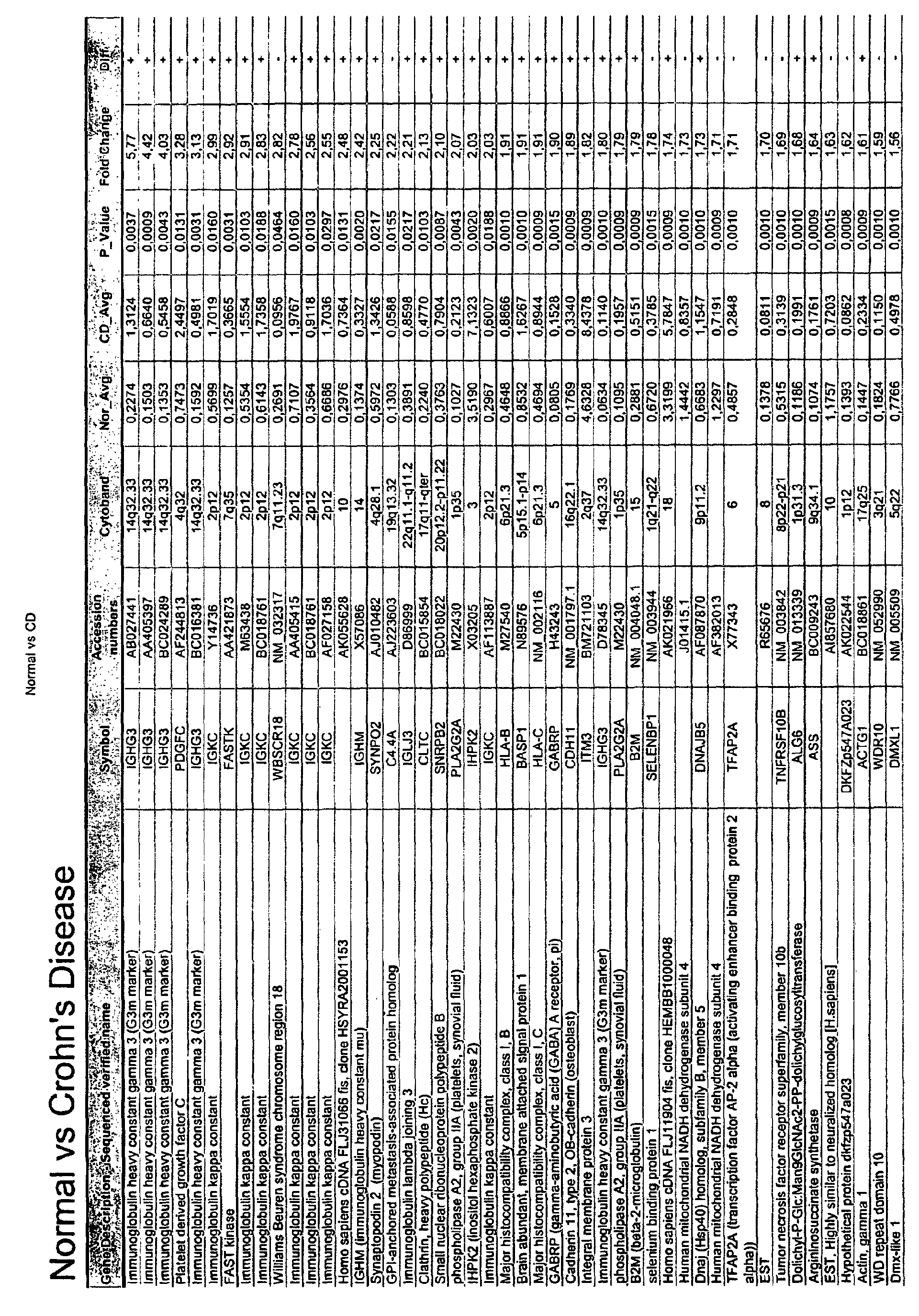

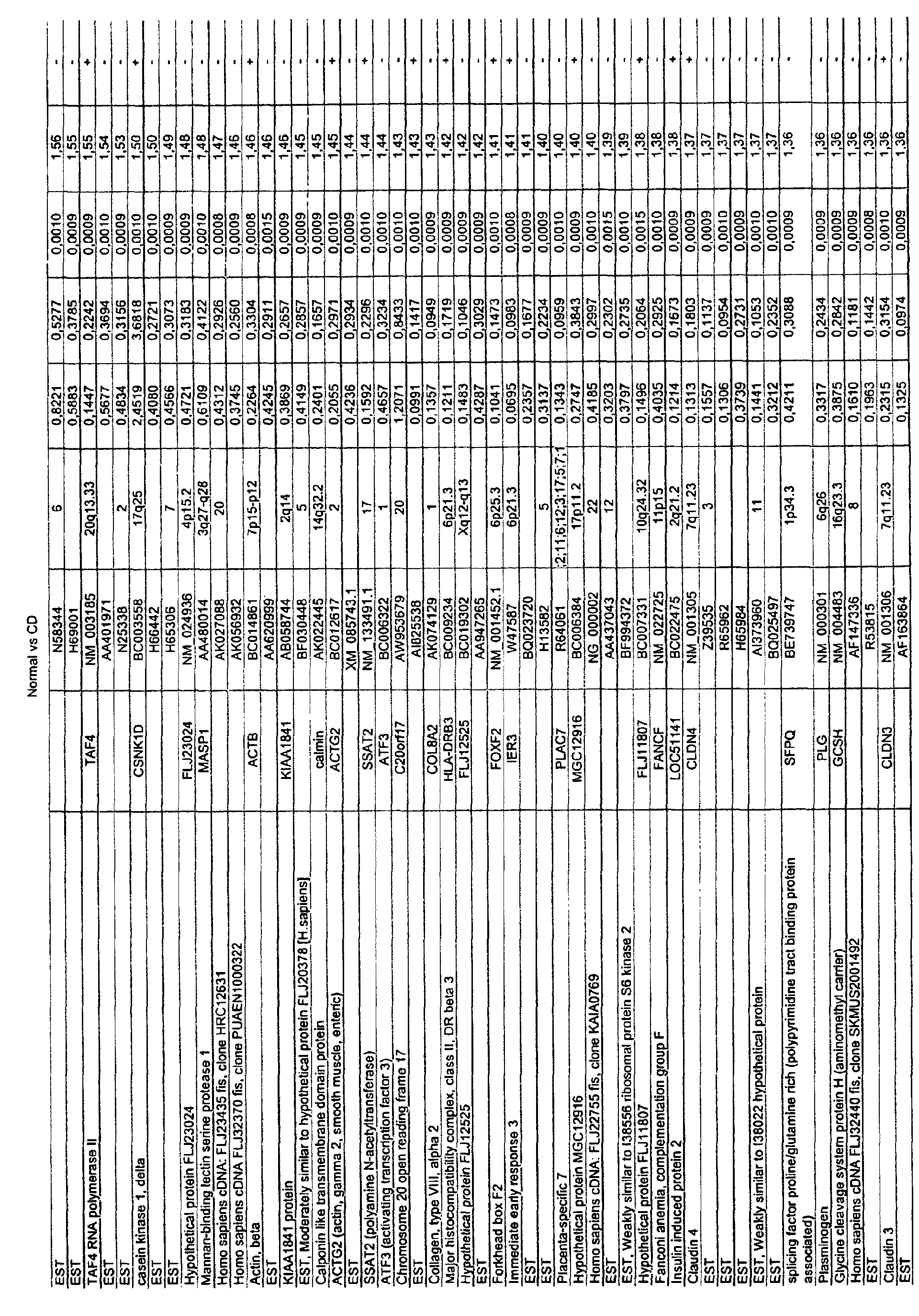

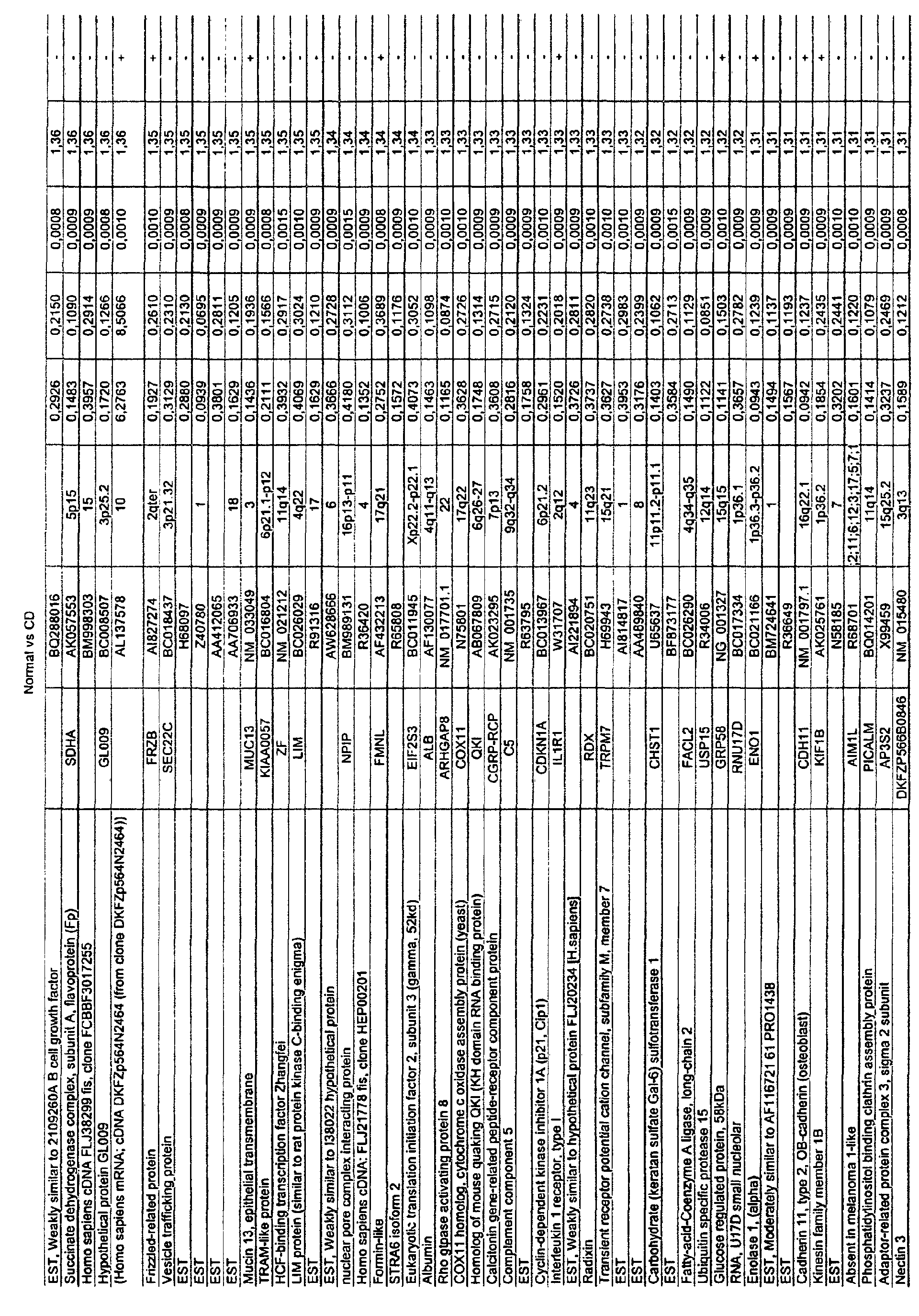

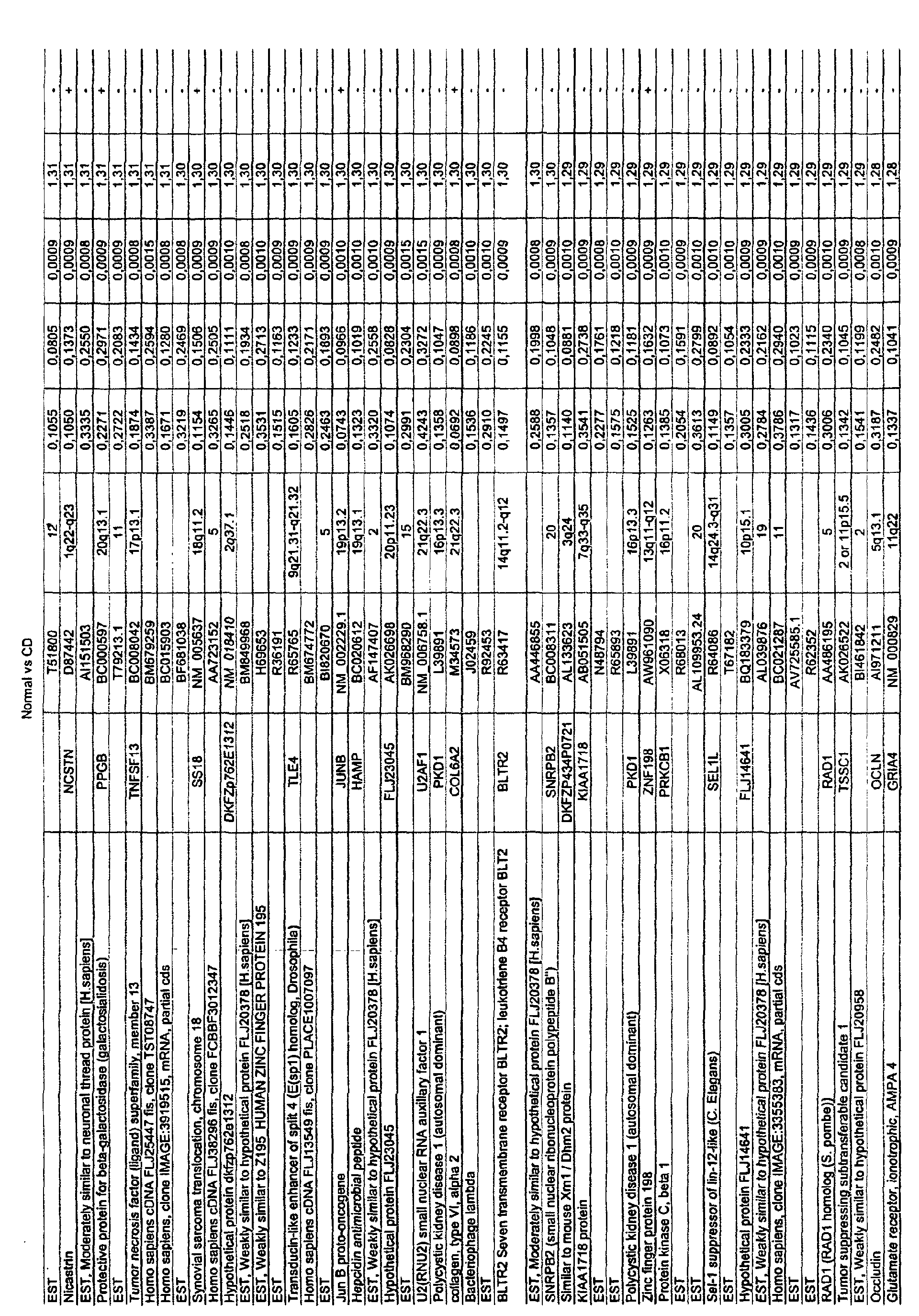

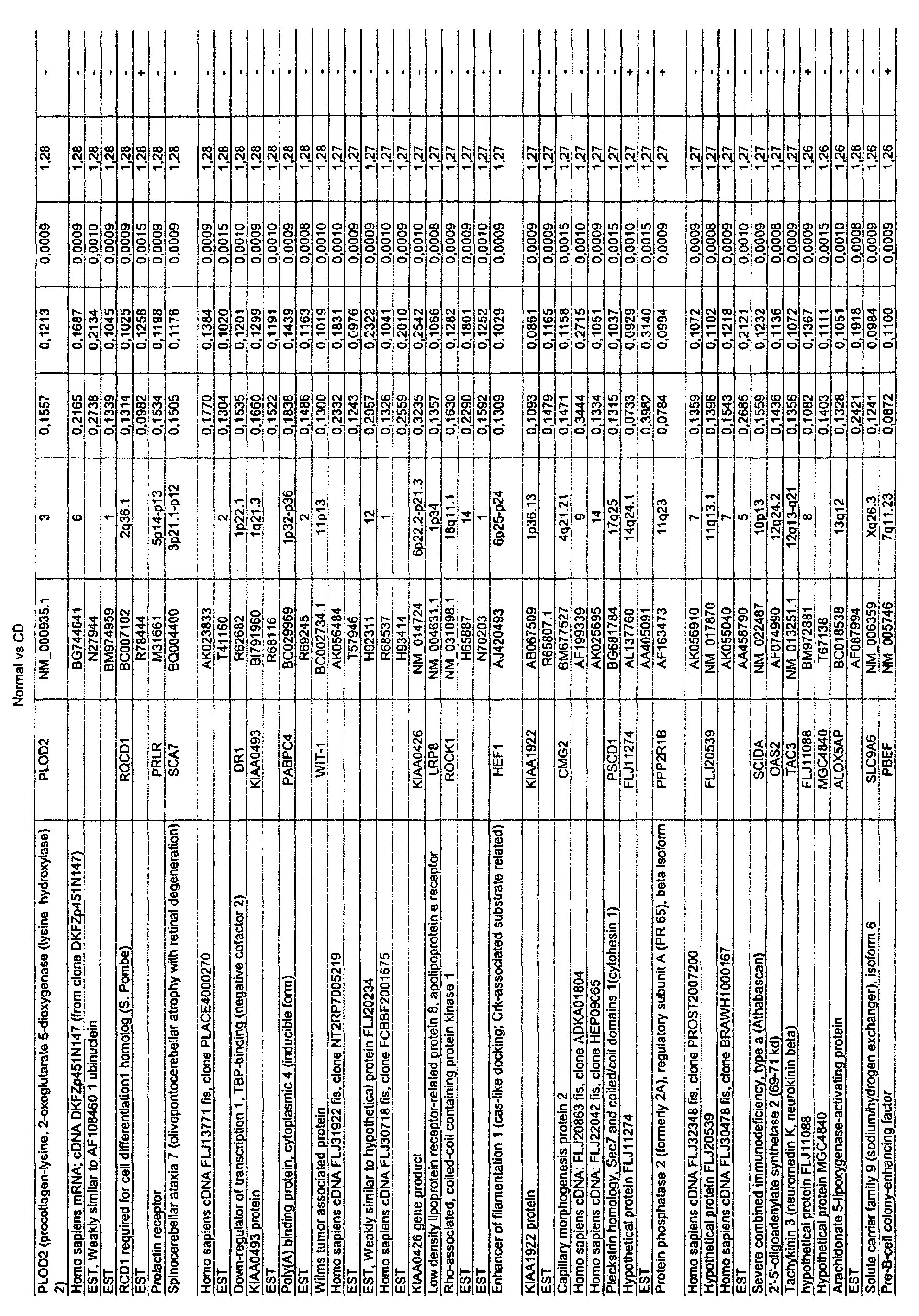

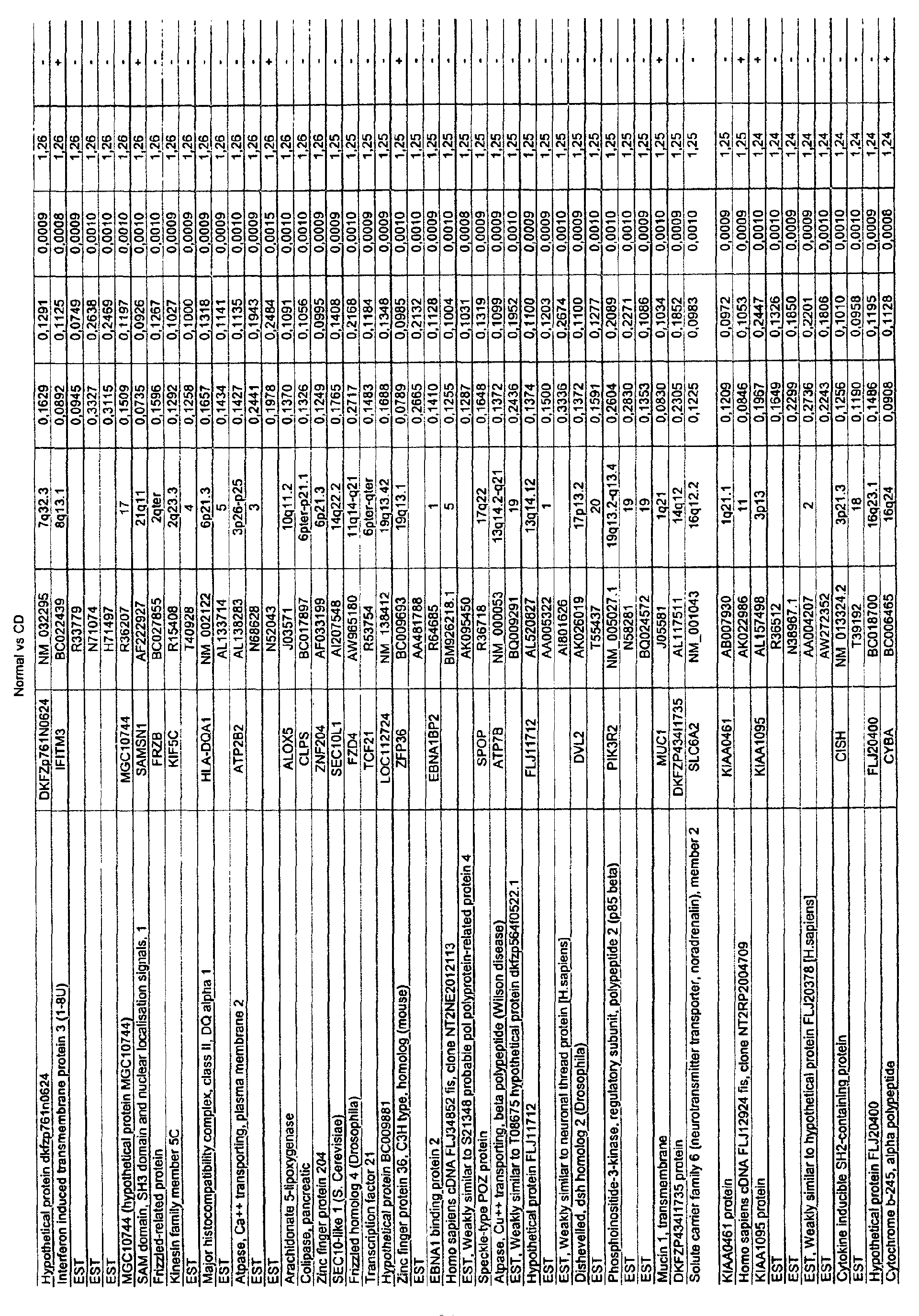

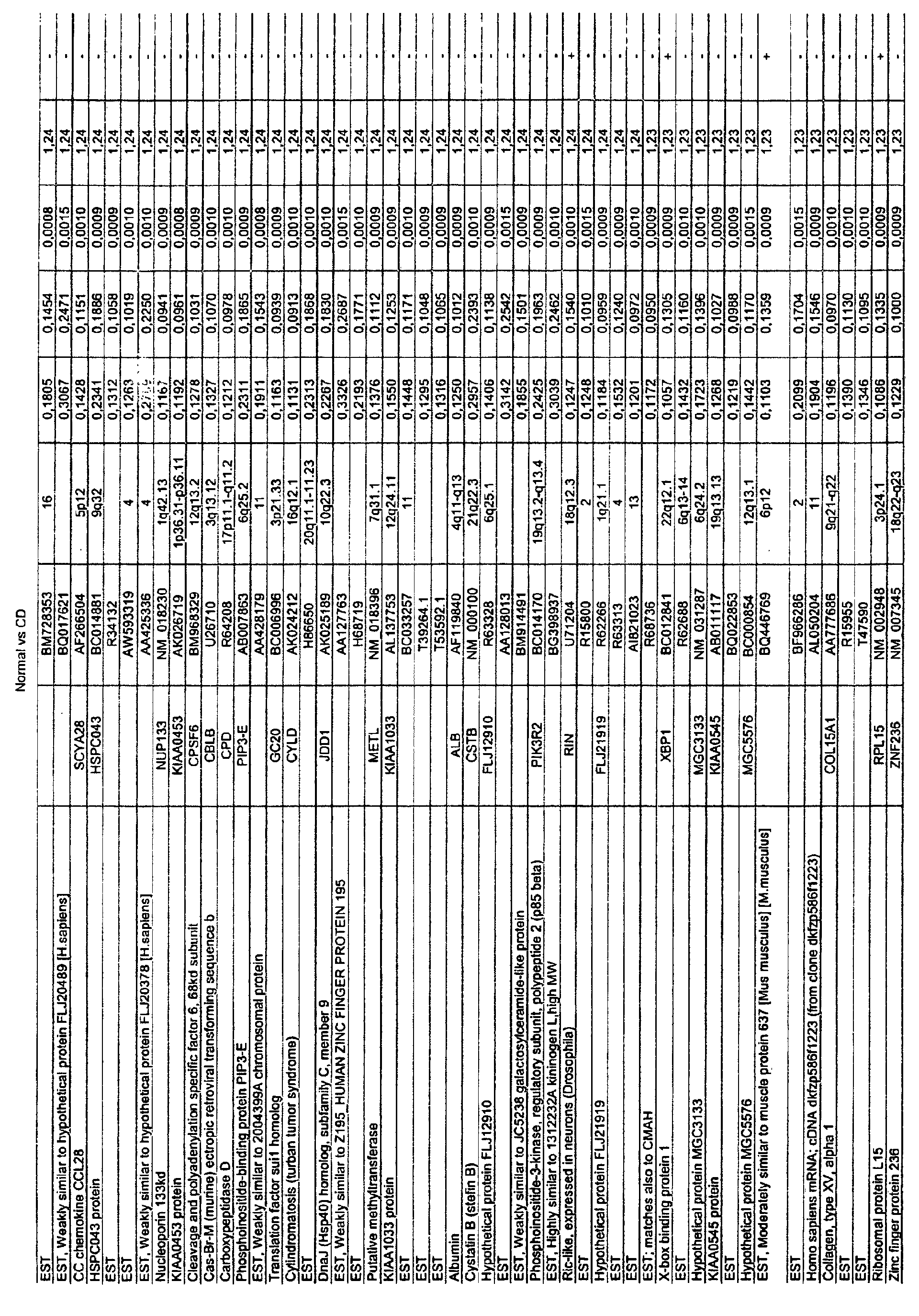

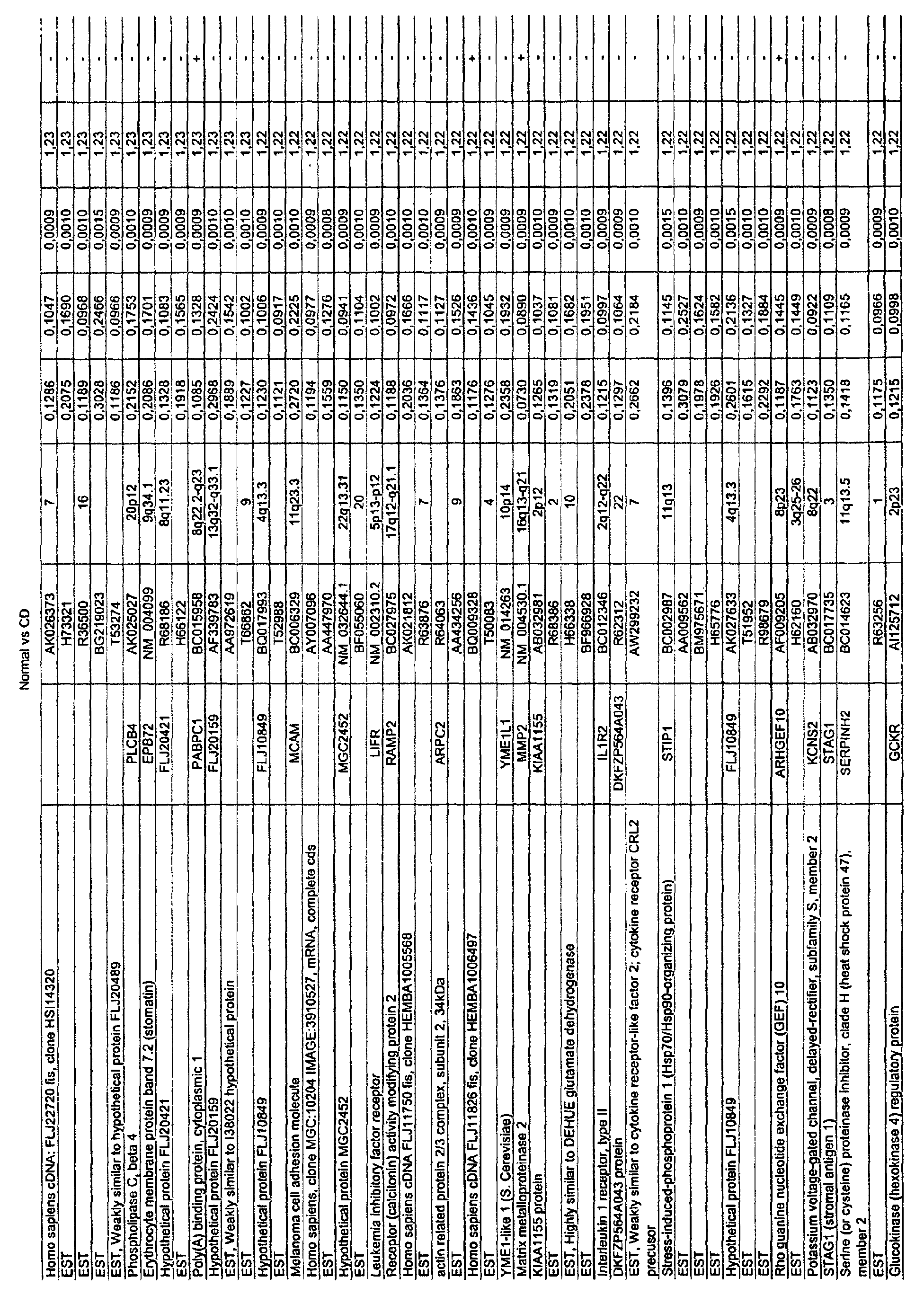

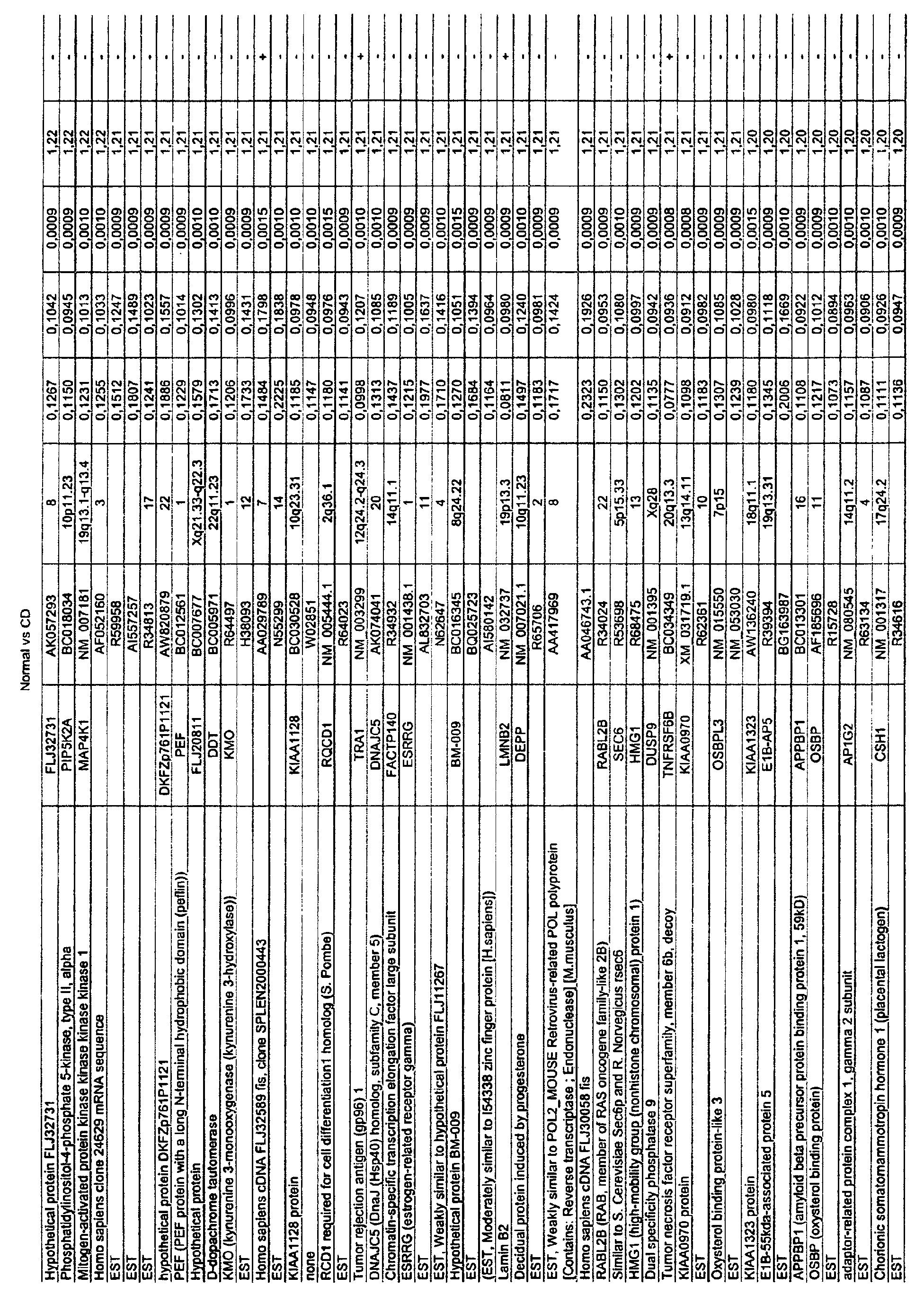

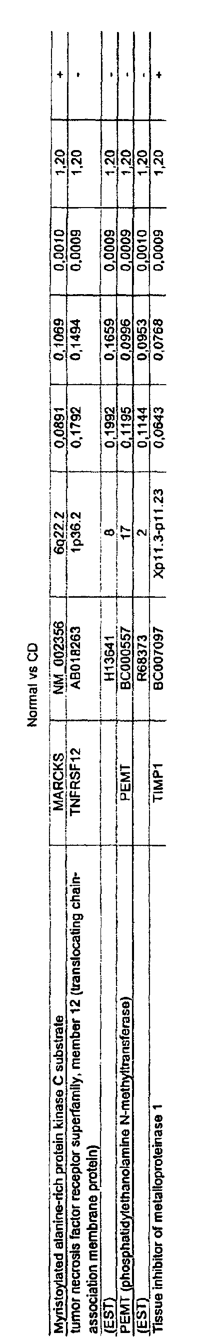

### (C) Verification of microarray results

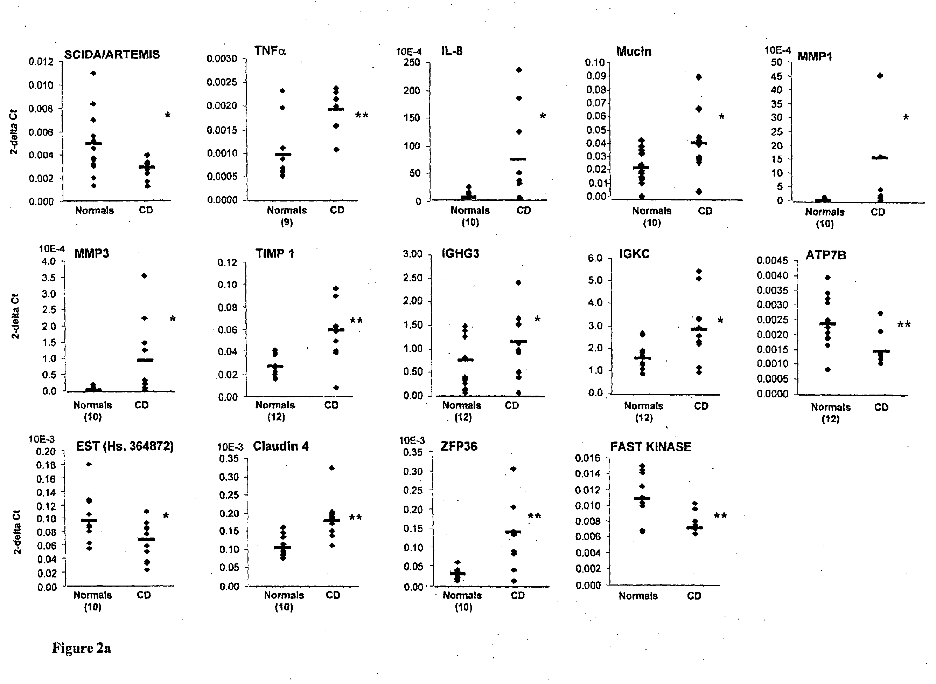

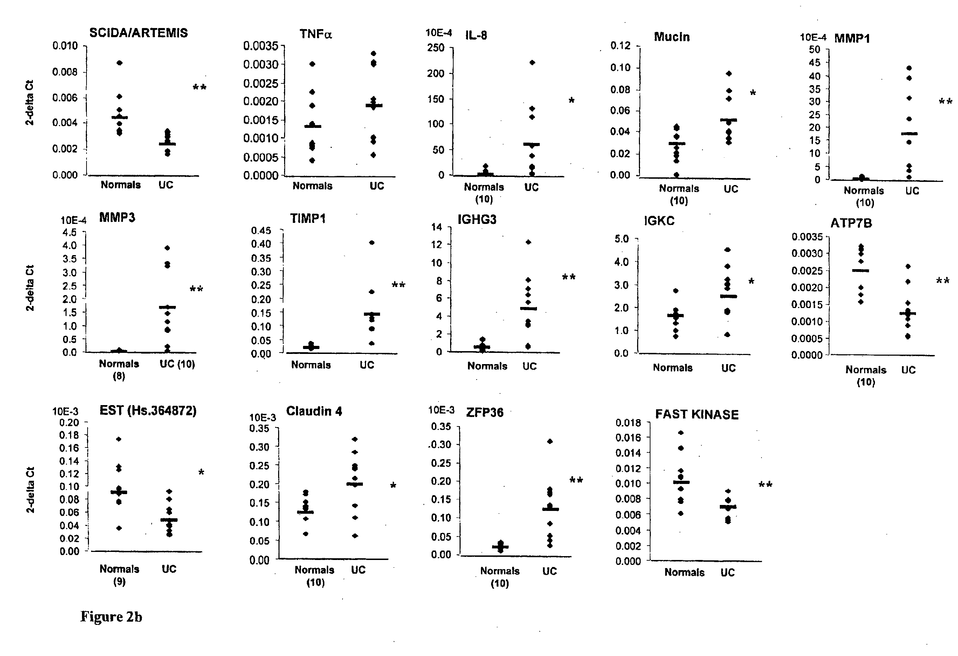

Real-time PCR using TaqMan technology on selected transcripts was carried out as a sensitive independent verification method of the array results (Figure 2: Normal to CD comparison; Figure 3: Normal to UC comparison). In a first step, small soluble cytokine mediators, that have previously been shown to be differentially regulated in disease, were used to benchmark the different sample populations. Results indicated that IL-8 was up-regulated in both CD (p=0.042) and UC (p=0.048) compared to controls, with TNF-α highly up-regulated in the CD population only (p=0.0013).

In the present study, array results showed that mucin 1 was significantly up-regulated in both forms of IBD, a finding that was confirmed by TaqMan analysis (CD: p=0.03; UC: p=0.02). Confirmation of the cluster of genes that showed the highest up-regulation between normal and IBD tissues, included members of the immunoglobulin family such as IGHG3 (CD: p=0.04, UC: p=0.003) and IGKC (CD: p=0.02, UC: p=0.003). Although MMP1 and MMP3 clones were up-regulated on the arrays, the p-values ranked below our cut-off criteria. However, TaqMan analysis showed both of these genes were significantly up-regulated in both forms of IBD with the differential up-regulation was more pronounced in UC samples (MMP1: CD: p=0.04, UC: p=0.005; MMP3: CD: p=0.05, UC: p=0.005). The up-regulation of TIMP1, which is the natural inhibitor of the matrix metalloproteinases, was also confirmed by TaqMan analysis (CD: p=0.004, UC: p=0.003). In both forms of IBD, ATPase ATP7B (CD: p=0.005, UC: p=0.002) and the SCIDA/ARTEMIS (CD: p=0.03, UC: p=0.006) gene were confirmed to be down-regulated. Other confirmed results included the up-regulation of claudin 4 (CD: p=0.003, UC: p=0.02) and the zinc finger protein 36 (ZFP36) ((CD: p=0.005, UC: p=0.002). With the sequence verified clone information, the confirmed result of one differentially regulated unknown EST is shown. Hs.29857 was shown to be down-regulated both on the arrays and in TaqMan analysis (CD: p=0.001, UC: p=0.002).

It is important to note that not all array results were confirmed by TaqMan. In the case of FAST kinase, this gene was significantly up-regulated in CD (by a factor of 2.9) and in UC (by a factor of 7.4) on the array. However, the TaqMan result consistently indicated that this gene was significantly down-regulated in both CD and UC (p < 0.005 in both cases). A possible reason for this result is that this gene has numerous splice variants, and may reflect cross-hybridisation effects between these variants on the array, which is not picked up by the more specific TaqMan method. In all, the confirmation rate was approximately 70% between the two methods, emphasising the importance of independent verification of array results.

### (D) Functional prediction of unknown ESTs

As an example of how relevant information can be obtained from unknown ESTs, some of the unannotated top differentially expressed genes between normals and IBD (see Table 6) were further analysed as described in Example 1. Two transcripts were in UniGene clusters with sequences homologous to structural proteins e.g. collagen (Hs.23921 & Hs.283848). The UniGene cluster Hs.24255 contains the vacuolar-sorting associated protein VPS13 (SOI1) and Hs.280858 shows a putative transmembrane phosphate acyltransferase domain. One EST (Hs.285519) was found to be situated in close proximity to a zinc finger protein 24 on chromosome 18 and could represent an alternately spliced 3' untranslated sequence of this potentially immune relevant gene. Additional gene products may function as an apoptosis regulator containing a B-cell lymphoma domain. (Hs.245326) and as a transcription factor with a zinc-binding sequence motif (Hs.278945). The protein sequence encoded by the mRNA assigned to the UniGene cluster Hs.36574 may contain a C-terminal AAI trypsin-alpha amylase inhibitor domain, which is also involved in phospholipid transfer. Another transcript (Hs.352537) mapped in close proximity to the 3' UTR of ADAM 12 (a disintegrin and metalloproteinase 12 (meltrin alpha)), which mediates several important processes such as TNF-α release, fertilisation and myboblast fusion. Hs.7159 may mediate phosphoinositide signalling due to the presence of a N-terminal pleckstrin homology domain.

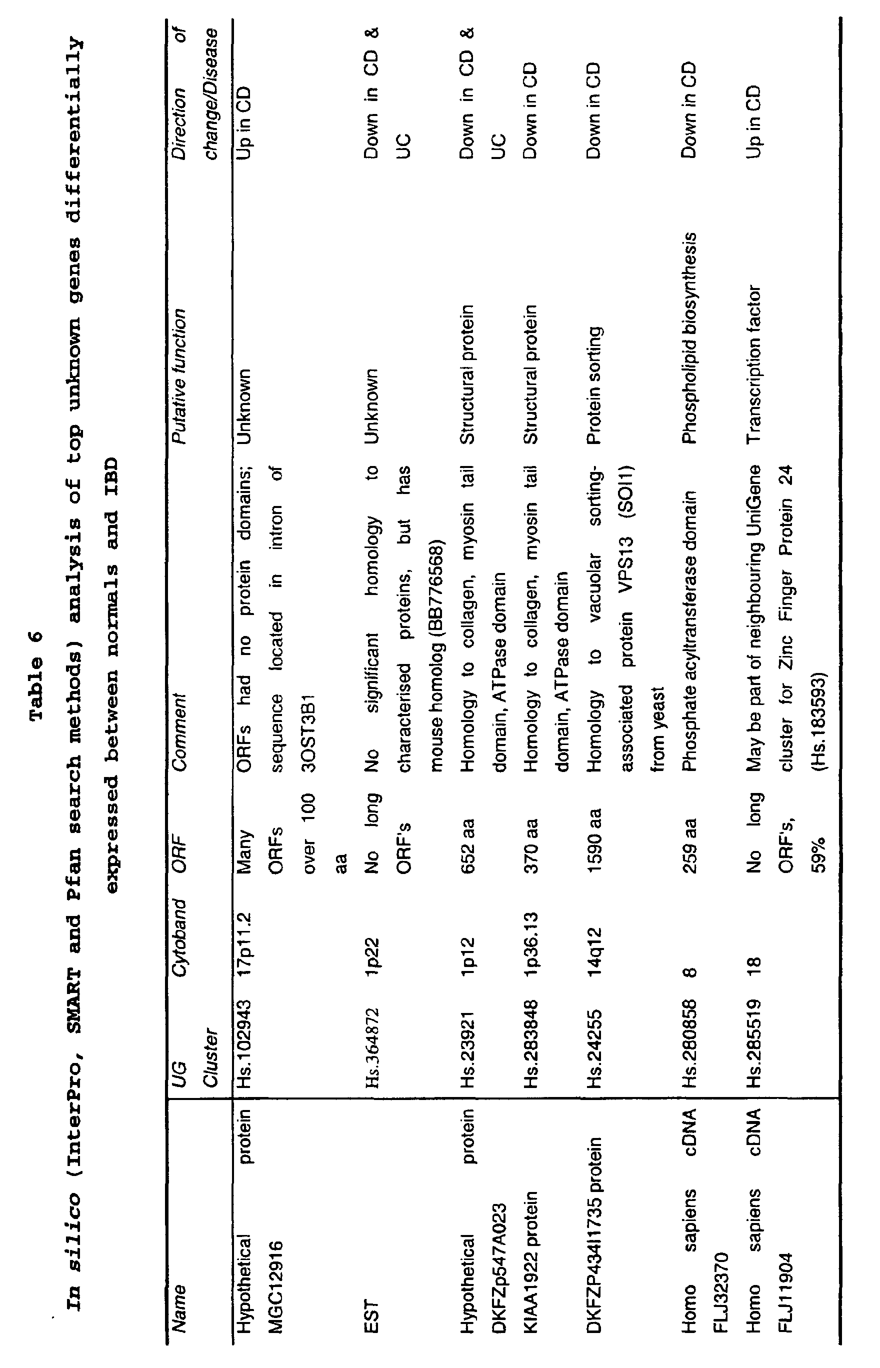

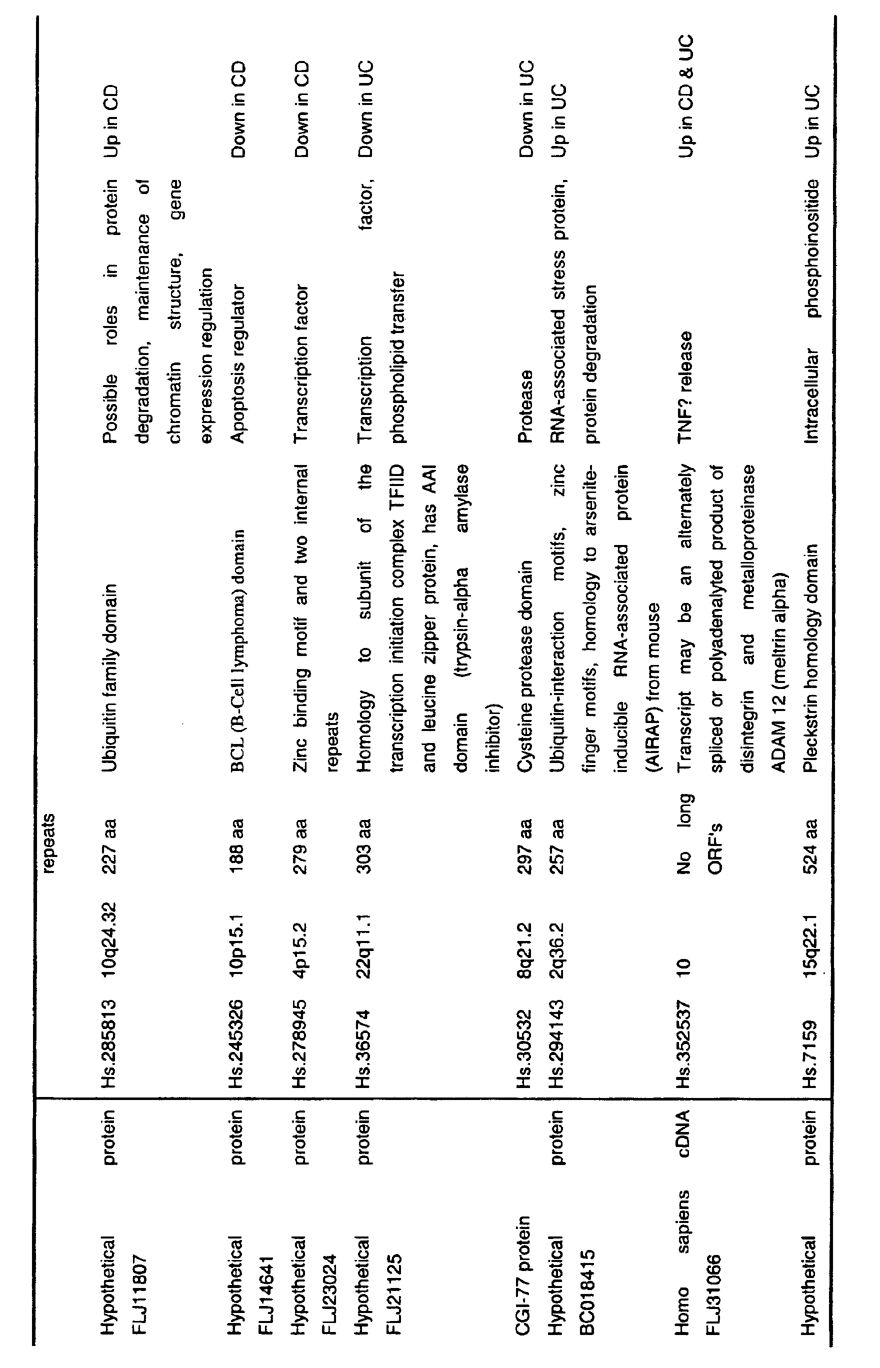

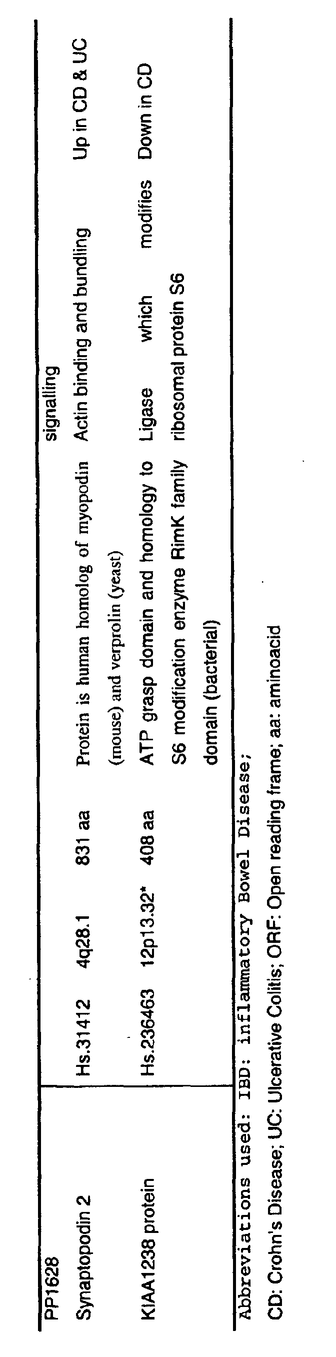

## Claims

1. A diagnostic composition comprising
(i)(a) at least one nucleic acid molecule which each comprises the nucleotide sequence of a gene listed in Table 4 or a fragment thereof and/or (b) at least one nucleic acid molecule which each comprises the nucleotide sequence of a gene listed Table 5 or a fragment thereof; or
(ii) at least one nucleic acid molecule which is capable of hybridizing to a nucleotide sequence of (i).

2. The diagnostic composition of claim 1 comprising
(i)(a) at least 50 nucleic acid molecules which each comprises the nucleotide sequence of a gene listed in Table 4 or a fragment thereof and/or (b) at least 50 nucleic acid molecules which each comprises the nucleotide sequence of a gene listed in Table 5 or a fragment thereof; or
(ii) at least 50 nucleic acid molecules which are capable of hybridizing to 50 different nucleotide sequences of (i).

3. The diagnostic composition of claim 1 comprising
(i)(a) at least 100 nucleic acid molecules which each comprises the nucleotide sequence of a gene listed in Table 4 or a fragment thereof and/or (b) at least 100 nucleic acid molecules which each comprises the nucleotide sequence of a gene listed in Table 5 or a fragment thereof; or
(ii) at least 100 nucleic acid molecules which are capable of hybridizing to 100 different nucleotide sequences of (i).

4. The diagnostic composition of claim 1 comprising
(i) (a) at least 200 nucleic acid molecules which each comprises the nucleotide sequence of a gene listed in Table 4 or a fragment thereof and/or (b) at least 200 nucleic acid molecules which each comprises the nucleotide sequence of a gene listed in Table 5 or a fragment thereof; or
(ii) at least 200 nucleic acid molecules which are capable of hybridizing to 200 different nucleotide sequences of (i).

5. The diagnostic composition of any one of claims 1 to 4, wherein the nucleic acid molecules are bound to a solid support.

6. The diagnostic composition of claim 5, wherein the solid support is a microarray.

7. The diagnostic composition of any one of claims 1 to 6, wherein the nucleic acid molecule has a length of at least 16 nucleotides.

8. Use of (a) nucleic acid molecule(s) as defined in any one of claims 1 to 7 for the preparation of a diagnostic composition for the diagnosis of inflammatory bowel disease or a related disease or a disposition to such a disease.

9. Use of (a) nucleic acid molecule(s) as defined in any one of claims 1(i), 2(i), 3(i), 4(i), or 5 to 7 for the preparation of a diagnostic composition for the diagnosis of Chrohn's disease (CD) or a disposition for such a disease.

10. Use of (a) nucleic acid molecule(s) as defined in any one of claims 1 (ii), 2 (ii), 3 (ii), 4 (ii), or 5 to 7 for the preparation of a diagnostic composition for the diagnosis of ulcerative colitis (UC) or a disposition to such a disease.

11. Use of (a) nucleic acid molecule(s) as defined in any one of claims 1 to 7 for the isolation or development of a compound useful for therapy or prevention of inflammatory bowel disease or a related disease.
